(19) Europäisches Patentamt
European Patent Office
Office européen des brevets

(11) EP 4 034 258 B1

(12) # EUROPÄISCHE PATENTSCHRIFT

(45) Veröffentlichungstag und Bekanntmachung des Hinweises auf die Patenterteilung:
**17.09.2025 Patentblatt 2025/38**

(21) Anmeldenummer: **20740300.7**

(22) Anmeldetag: **13.07.2020**

(51) Internationale Patentklassifikation (IPC):
***A61Q 5/06*** (2006.01) ***A61K 8/92*** (2006.01)
***A61K 8/898*** (2006.01) ***A61K 8/39*** (2006.01)
***A61K 8/86*** (2006.01)

(52) Gemeinsame Patentklassifikation (CPC):
**A61Q 5/065; A61K 8/39; A61K 8/86; A61K 8/898; A61K 8/922**

(86) Internationale Anmeldenummer:
**PCT/EP2020/069764**

(87) Internationale Veröffentlichungsnummer:
**WO 2021/058158 (01.04.2021 Gazette 2021/13)**

(54) **MITTEL ZUM FÄRBEN VON KERATINISCHEM MATERIAL MIT AMINOSILIKON, FARBGEBENDER VERBINDUNG UND ETHOXYLIERTEM FETTSÄUREESTER**

AGENT FOR DYEING KERATINOUS MATERIAL WITH AMINOSILICONE, COLORANT COMPOUND AND ETHOXYLATED FATTY ACID ESTER

AGENT POUR LA TEINTURE DE MATIÈRES KÉRATINIQUES AVEC UNE AMINOSILICONE, UN COMPOSÉ COLORANT ET UN ESTER D'ACIDE GRAS ÉTHOXYLÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.09.2019 DE 102019214466**

(43) Veröffentlichungstag der Anmeldung:
**03.08.2022 Patentblatt 2022/31**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **MOCH, Melanie**
  **41542 Dormagen (DE)**
- **HILBIG, Sandra**
  **44894 Bochum (DE)**
- **KESSLER-BECKER, Daniela**
  **51371 Leverkusen (DE)**

(56) Entgegenhaltungen:
**WO-A1-2015/090804 WO-A1-2018/206453**
**WO-A2-2014/044602 DE-A1- 102014 221 536**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 034 258 B1

## Beschreibung

Gegenstand der vorliegenden Anmeldung ist ein Mittel zum Färben von keratinischem Material, insbesondere menschlichen Haaren, welches mindestens ein aminofunktionalisiertes Silikonpolymer (a1), das Struktureinheiten der Formel (Si-I) und der Formel (Si-II) umfasst, mindestens eine farbgebende Verbindung aus der Gruppe der Pigmente (a2) sowie mindestens ein Anlagerungsprodukt von Ethylenoxid an Ester gebildet aus $C_{12}$-$C_{24}$-Fettsäuren und aliphatischen $C_1$-$C_{12}$-Alkoholen (a3) enthält.

Ein zweiter Gegenstand dieser Anmeldung ist ein Verfahren zum Färben von keratinischem Material, insbesondere menschlichen Haaren, wobei ein Mittel des ersten Erfindungsgegenstands auf dem keratinischen Material angewendet, einwirken gelassen und danach wieder mit Wasser ausgewaschen wird.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

WO 2015/090804 A1 betrifft ein wasserhaltiges Mittel zum Färben von Keratinfasern, welches einen pH-Wert von 1,0 bis 5,5 besitzt und welches ein Aminosilikon und einen direktziehenden Säurefarbstoff enthält.

Gegenstand der Anmeldung WO 2014/044602 A2 sind Mittel zum Behandeln von Keratinmaterial, enthaltend eine Säure (a), einen direktziehenden Farbstoff (b) und ein Aminosilikon.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

In DE 10 2014 221 536 A1 werden Mittel zur temporären Farbveränderung von keratinischen Fasern beschrieben, die mindestens 20 Gew.-% C2-C8-Alkohole (a), mindestens ein Farbpigment (b) und mindestens ein nichtionisches, polyalkoxyliertes Silikon (c) enthalten.

WO 2018/206453 A1 betrifft Zusammensetzungen zum Färben von Keratinfasern, enthaltend mindestens ein Copolymer aus Crotonsäure und Vinylester (i), ein Silikon (ii) und ein Pigment (iii).

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Vor allem die Farbintensitäten und Waschechtheiten von Färbemitteln, die auf dem Einsatz von Pigmenten basieren, sind noch stark verbesserungswürdig.

Es war die Aufgabe der vorliegenden Erfindung, ein Färbesystem bereitzustellen, das mit der oxidativen Färbung nach Möglichkeit vergleichbare Farbintensitäten besitzt. Hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie insbesondere Pigmente) in extrem dauerhafter Weise auf den Haaren zu fixieren. Bei Anwendung der Mittel in einem Färbeverfahren sollten besonders intensive Färbeergebnisse mit guten Echtheitseigenschaften erzielt werden. Darüberhinaus sollten die Mittel auch eine verbesserte Grauabdeckung besitzen.

Überraschenderweise hat sich nun herausgestellt, dass die vorgenannte Aufgabe hervorragend gelöst werden kann, wenn keratinische Materialien, insbesondere Haare, mit einem Mittel gefärbt werden, welches mindestens ein aminofunktionalisiertes Silikonpolymer (a1), das Struktureinheiten der Formel (Si-I) und der Formel (Si-II) umfasst, mindestens eine farbgebende Verbindung aus der Gruppe der Pigmente (a2), und mindestens ein Anlagerungsprodukt von Ethylenoxid an Ester gebildet aus $C_{12}$-$C_{24}$-Fettsäuren und aliphatischen $C_1$-$C_{12}$-Alkoholen (a3) enthält.

Ein erster Gegenstand der vorliegenden Erfindung ist ein Mittel zum Färben von keratinischem Material, insbesondere menschlichen Haaren, enthaltend

(a1) mindestens ein aminofunktionalisiertes Silikonpolymer, das Struktureinheiten der Formel (Si-I) und der Formel (Si-II) umfasst

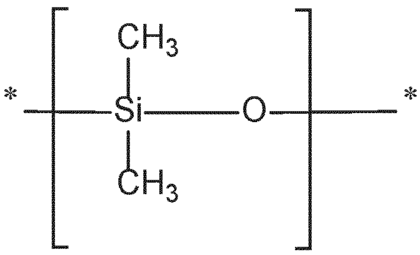
(Si-I)

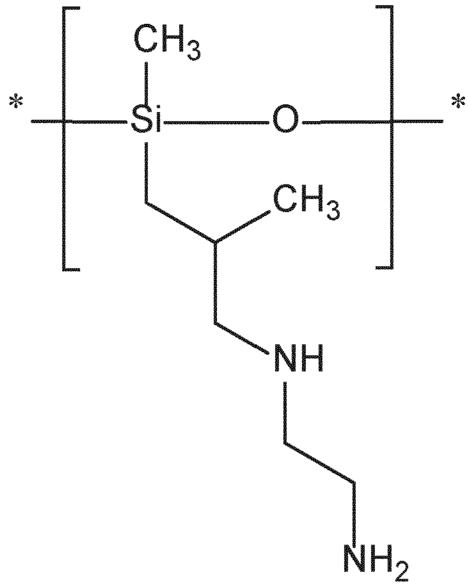
(Si-II),

und
(a2) mindestens eine farbgebende Verbindung aus der Gruppe der Pigmente, und
(a3) mindestens ein Anlagerungsprodukt von Ethylenoxid an Ester gebildet aus $C_{12}$-$C_{24}$-Fettsäuren und aliphatischen $C_1$-$C_{12}$-Alkoholen

Im Zuge der zu dieser Erfindung durchgeführten Arbeiten hat sich überraschenderweise gezeigt, dass der Einsatz eines ethoxylierten Fettsäure-Esters (a3) in einem Mittel, welches das Aminosilikon (a1) sowie einPigment (a2) enthält, zu einer Verbesserung der Farbintensität führt, wenn dieses Mittel in einem Färbeverfahren auf dem keratinischen Material, insbesondere auf menschlichen Haaren, angewendet wird.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.
Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Mittel zur Färbung

Der Begriff "Mittel zur Färbung" wird im Rahmen dieser Erfindung für eine durch Einsatz von farbgebenden Verbindungen, insbesondere Pigmenten, hervorgerufene Farbgebung des Keratinmaterials, insbesondere des Haares, verwendet. Bei dieser Färbung lagern sich die Pigmente als farbgebende Verbindungen in einem besonders homogenen, gleichmäßigen und glatten Film an der Oberfläche des Keratinmaterials ab.

aminofunktionalisierte Silikonpolymere (a1)

Als ersten erfindungswesentlichen Inhaltsstoff (a1) enthält das Mittel mindestens ein aminofunktionalisiertes Silikonpolymer, das Struktureinheiten der Formel (Si-I) und der Formel (Si-II) umfasst. Das aminofunktionalisiertes Silikonpolymer kann alternativ auch als Aminosilikon oder Amodimethicone bezeichnet werden.
Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.
Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der

vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Färbungen mit den allerbesten Waschechtheiten konnten erhalten werden, wenn im erfindungsgemäßen Verfahren mindestens ein Mittel (a) auf dem keratinischen Material appliziert wurde, das mindestens ein aminofunktionalisiertes Silikonpolymer (a1) enthält, das Struktureinheiten der Formel (Si-I) und der Formel (Si-II) umfasst

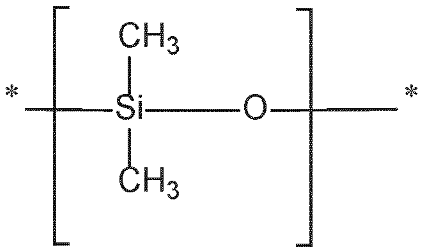

(Si-I)

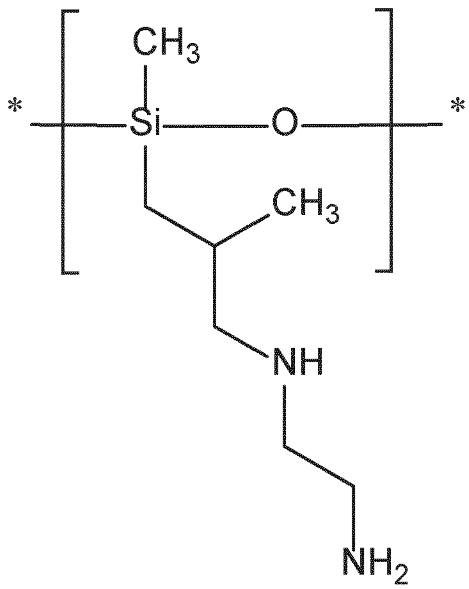

(Si-II).

Das erfindungsgemäße Mittel ist dadurch gekennzeichnet, dass es mindestens ein aminofunktionalisiertes Silikonpolymer (a1) enthält, das Struktureinheiten der Formel (Si-I) und der Formel (Si-II) umfasst

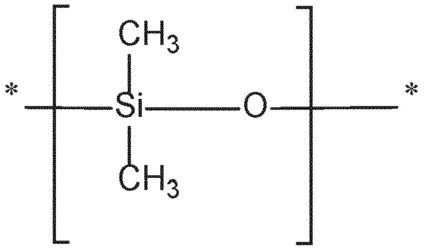
(Si-I)
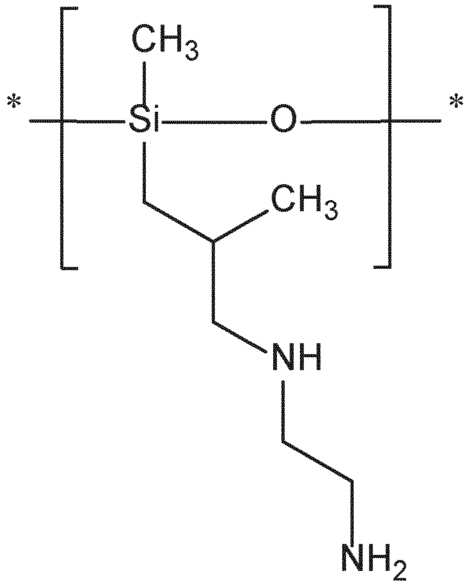
(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.
Es hat sich als besonders vorteilhaft herausgestellt, wenn das erfindungsgemäße Mittel das oder die aminofunktionalisierten Silikonpolymere (a1) in bestimmten Mengenbereichen enhält. Besonders gute Ergebnisse konnten erhalten werden, wenn das Mittel - bezogen auf das Gesamtgewicht des Mittels - in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.
Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

farbgebende Verbindungen (a2)

Als zweiten wesentlichen Bestandteil enthält das erfindungsgemäße Mittel mindestens eine farbgebende Verbindung aus der Gruppe der Pigmente (a2).
Unter farbgebenden Verbindungen werden im Sinne der Erfindung Substanzen verstanden, die in der Lage sind, dem Keratinmaterial eine Färbung zu verleihen. Die farbgebenden Verbindungen werden ausgewählt aus der Gruppe der Pigmente Farbstoffe.
Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.
Geeignete Farbpigmente können anorganischen und/oder organischen Ursprungs sein.
In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.
Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens eine farbgebende Verbindung (a2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA

Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch ein oder mehrere farbgebende Verbindungen (a2) aus der Gruppe der organischen Pigmente enthalten
Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einerweiteren besonders bevorzugten Ausführungsform ist ein erfindungsgemäßes Mittel dadurch gekennzeichnet, dass es mindestens eine farbgebende Verbindungen (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (a) ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Die farbgebenden Verbindungen (a2) aus der Gruppe der Pigmente, stellen den zweiten wesentlichen des erfindungsgemäßen Mittels dar und werden bevorzugt in bestimmten Mengenbereichen im Mittel eingesetzt.

Besonders gute Ergebnisse wurden erhalten, wenn das Mittel - bezogen auf das Gesamtgewicht des Mittels - ein

oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Ethoxylierte Fettsäure-Ester (a3)

Als dritten wesentlichen Inhaltsstoff (a3) enthalten die erfindungsgemäßen Mittel mindestens ein Anlagerungsprodukt von Ethylenoxid an Ester gebildet aus $C_{12}$-$C_{24}$-Fettsäuren und aliphatischen $C_1$-$C_{12}$-Alkoholen.

Die Anlagerungsprodukte von Ethylenoxid an die Ester gebildet aus $C_{12}$-$C_{24}$-Fettsäuren und aliphatischen $C_1$-$C_{12}$-Alkoholen (a3) können kurz auch als ethoxylierte Fettsäureester (a3) bezeichnet werden.

Die ethoxylierten Fettsäure-Ester (a3) basieren auf $C_{12}$-$C_{24}$-Fettsäuren. Bei diesen erfindungsgemäßen $C_{12}$-$C_{24}$-Fettsäuren handelt es sich um lineare oder verzweigte, gesättigte oder einfach oder mehrfach ungesättigte Fettsäuren, die auch eine oder mehrere Hydroxygruppen tragen können. Die erfindungsgemäßen $C_{12}$-$C_{24}$-Fettsäuren sind dadurch gekennzeichnet, dass die 12 bis 24 Kohlenstoffatome umfassen. Weiterhin tragen die $C_{12}$-$C_{24}$-Fettsäuren mindestens eine Carbonsäure-Gruppierung. Diese Carbonsäure-Gruppierung bildet entweder mit den aliphatischen $C_1$-$C_{12}$-Alkoholen einen Ester aus, oder aber sie bildet ein Addukt mit Ethylenoxid, wobei dieses Addukt dann mit einem aliphatischen C1-C12-Alkoholen weiter reagiert.

Zur Ausbildung der erfindungsgemäßen ethoxylierten Fettsäureester (a3) können beispielsweise eine oder mehrere Fettsäuren eingesetzt werden, die ausgewählt sind aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure], Nervonsäure [(15Z)-Tetracos-15-ensäure] und/oder Rizinusölsäure ((9Z,12*R*)-12-Hydroxy-9-octadecensaeure.

Die ethoxylierten Fettsäure-Ester (a3) stellen Anlagerungsprodukte von Ethylenoxid an die Ester der zuvor beschriebenen $C_{12}$-$C_{24}$-Fettsäuren und aliphatischen $C_1$-$C_{12}$-Alkoholen dar. Kennzeichnend für die $C_1$-$C_{12}$-Alkohole ist, dass sie 1 bis 12 Kohlenstoffatome umfassen. Die Alkohole sind aliphatisch und können linear oder verzweigt, gesättigt oder einfach oder mehrfach ungesättigt sein. Die entsprechenden $C_1$-$C_{12}$-Alkohole können ein- oder mehrwertige Alkohole sein, d.h. die Alkohole können eine oder mehrere Hydroxygruppen besitzen.

Einwertige $C_1$-$C_{12}$-Alkohole besitzen eine Hydroxygruppe. Als geeignete Vertreter können beispielsweise Methanol, Ethanol, n-Propanol, Iso-Propanol, n-Butanol, n-Pentanol, n-Hexanol, n-Octanol, n-Decanol und n-Dodecanol genannt werden.

Zweiwertige $C_1$-$C_{12}$-Alkohole besitzen zwei Hydroxygruppen. Als geeignete Vertreter können beispielsweise 1,2-Ethandiol, 1,2-Propandiol, 1,3-Propandiol, 1,4-Butandiol, 1,5-Pentandiol, 1,2-Hexandiol, 1,6-Hexandiol, 1,2-Octandiol, 1,8-Octandiol genannt werden.

Werden zweiwertige $C_1$-$C_{12}$-Alkohole zur Ausbildung der Ester eingesetzt, so können eine oder beide Hydroxygruppe eine Ester-Gruppe mit dem oder den $C_{12}$-$C_{24}$-Fettsäuren ausbilden.

An die freie Hydroxygruppe kann sich auch ein oder mehrere Mole Ethylenoxid anlagern.

Dreiwertige $C_1$-$C_{12}$-Alkohole besitzen drei Hydroxygruppen. Als geeigneter Vertreter kann beipsielsweise Glycerin genannt werden.

Werden dreiwertige $C_1$-$C_{12}$-Alkohole zur Ausbildung der Ester eingesetzt, so können eine, beide oder alle drei Hydroxygruppe eine Ester-Gruppe mit dem oder den $C_{12}$-$C_{24}$-Fettsäuren ausbilden.

An die freie(n) Hydroxygruppe(n) können sich auch ein oder mehrere Mole Ethylenoxid anlagern.

Ein entsprechendes Anlagerungsprodukt von Ethylenoxid an die Ester aus $C_{12}$-$C_{24}$-Fettsäuren und aliphatische $C_1$-$C_{12}$-Alkoholen (a3) entsteht, wenn die $C_{12}$-$C_{24}$-Fettsäure selbst oder der bereits aus ihr ausgebildete Ester mit Ethylenoxid (Alternativanme 1,2-Epoxyethan, CAS-Nummer 75-21-8) umgesetzt wird.

Wird die $C_{12}$-$C_{24}$-Fettsäure selbst mit Ethylenoxid umbesetzt, so kann sich zunächst ein Addukt ausgehend von der Carbonsäure-Gruppierung der $C_{12}$-$C_{24}$-Fettsäure und dem Ethylenoxid ausbilden, so dass eine Gruppierung *-C(O)-O-$CH_2$-$CH_2$-O-* entsteht. Bei dieser Gruppierung handelt es sich ebenfalls um einen Ester. Wird pro Mol Fettsäure ein Mol Ethylenoxid umgesetzt, so bildet sich im Mittel ein einfaches Addukt mit einer Einheit *-CH2-CH2-O-* aus. Abhängig davon, in welchem molaren Überschuss das Ethylenoxid eingesetzt wird, können sich jedoch auch mehrfache Addukte ausbilden, wobei pro Mol $C_{12}$-$C_{24}$-Fettsäure mehrere Einheiten *-CH2-CH2-O-* vorliegen.

Zur Ausbildung des Esters (a3) kann dieses Addukt dann weiterhin mit mindestens einem $C_1$-$C_{12}$-Alkohol

umgesetzt werden.

Die mit einem Stern gekennzeichneten Positionen stellen hierbei die Bindung zum restlichen Teil der Fettsäure und die Bindung mit dem restlichen Teil des Alkohols dar.

Formal stellt sich dieser Typ von Anlagerungsprodukten von Ethylenoxid an die Ester aus $C_{12}$-$C_{24}$-Fettsäuren und aliphatischen $C_1$-$C_{12}$-Alkoholen (a3) damit so dar, dass sich eine oder mehrere Einheiten $-O-CH_2-CH_2-$ zwischen der Carbonylgruppe der Fettsäure und dem Sauerstoffatom des Alkohols befinden.

Ein weiterer Typ von Anlagerungsprodukten von Ethylenoxid an die Ester aus $C_{12}$-$C_{24}$-Fettsäuren und aliphatische $C_1$-$C_{12}$-Alkohole (a3) liegt dann vor, wenn ein Ester mit Ethylenoxid umgesetzt wird, wobei dieser Ester durch Veresterung einer $C_{12}$-$C_{14}$-Fettsäure mit mindestens einer Hydroxygruppe mit einem $C_1$-$C_{12}$-Alkohol erhalten wurde. Beispielsweise kann Rizinusölsäure ((9Z,12R)-12-Hydroxy-9-octadecensaeure) zunächst mit Methanol , Ethanol oder Glycerin verestert werden. Bei Ausbildung dieser Addukte lagert sich nun das Ethylenoxid an die Hydroxygruppe der Rizinusölsäure an und/oder an die noch freien Hydroxygruppen des Glycerins an.

Wird pro Mol Fettsäure ein Mol Ethylenoxid umgesetzt, so kann sich zum Beipsiel eine Einheit *-CH2-CH2-O-* an die Hydroyxgruppe der Rizinusölsäure addieren. Abhängig davon, in welchem molaren Überschuss das Ethylenoxid eingesetzt wird, können sich jedoch auch mehrfache Addukte ausbilden, wobei an jede Hydroxygruppe der Fettsäure mehrere Einheiten $*-CH_2-CH_2-O-*$ addiert wurden, und/oder wobei auch an jede freie Hydroxygruppe des Glycerins mehrere Einheiten $*-CH_2-CH_2-O-*$ addiert wurden.

Zur Lösung der erfindungsgemäßen Aufgabenstellung hat sich der Einsatz von ganz bestimmten $C_1$-$C_{12}$-Alkoholen zur Ausbildung des ethoxylierten Fettsäure-Esters (a3) als ganz besonders gut geeignt erwiesen. Besonders gute Effekte konnten beobachtet, wenn mindestens ein Anlagerungsprodukt von Ethylenoxid an einen Ester enthält, der durch Veresterung von einer, zwei oder drei $C_{12}$-$C_{24}$-Fettsäuren mit Glycerin erhalten wurde.

Im Rahmen einer weiteren ganz besonders bevorzugten Ausführungsform ist ein erfindungsgemäßes Mittel dadurch gekennzeichnet, dass es

(a3) mindestens ein Anlagerungsprodukt von Ethylenoxid an einen Ester enthält, der durch Veresterung von einer, zwei oder drei $C_{12}$-$C_{24}$-Fettsäuren mit Glycerin erhalten wird.

Eine ganz besonders gut geeignete Substanzgruppe der ethoxylierten Fettsäureester (a3) stellen die Verbindungenn der allgemeinen Formel (EFA-I) dar.

Im Rahmen einer weiteren ganz besonders bevorzugten Ausführungsform ist ein erfindungsgemäßes Mittel dadurch gekennzeichnet, dass es

(a3) mindestens einen ethoxylierten Fettsäureester der allgemeinen Formel (EFA-I) enthält

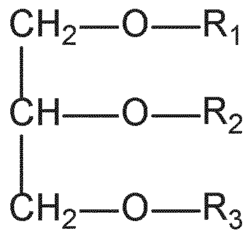

(EFA-I)

wobei

R1, R2, R3 unabhängig voneinander für eine Gruppierung $-(CH_2-CH_2-O)_v-H$, oder für eine Gruppierung der allgemeinen Formel (EFA-II) stehen,

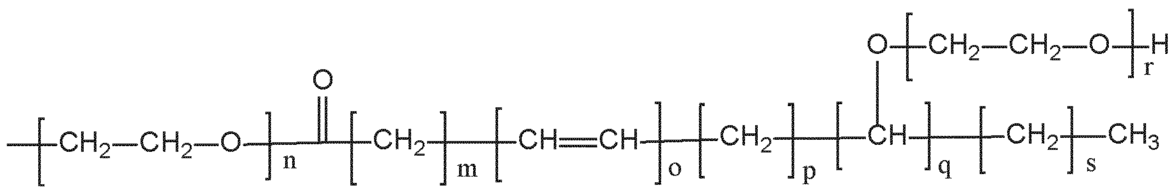

(EFA-II),

wobei

- v für eine ganze Zahl von 0 bis 100 steht,
- n für eine ganze Zahl von 0 bis 100 steht für eine ganze Zahl von 1 bis 8 steht,
- m o für eine ganze Zahl von 0 bis 3 steht,

p für eine ganze Zahl von 1 bis 8 steht,
q für die Zahl 0 oder 1 steht,
r für eine ganze Zahl von 1 bis 100, und
s für eine ganze Zahl von 1 bis 8 steht,

mit der Maßgabe, dass

- mindestens einer der Reste R1, R2, R3 für eine Gruppierung der allgemeinen Formel (EFA-II) steht, und dass
- mindestens eine der Indexzahlen für v, n und/oder q für eine Zahl ungleich 0 stehen.

Die Verbindungen der Formel (EFA-I) basieren auf dem $C_1$-$C_{12}$-Alkohol Glycerin, welcher die Reste R1, R2 und R3 trägt.

Die Reste R1, R2 und R3 können unabhängig voneinander gewählt werden. Auch die Substituenten in den Resten R1, R2 und R3 können jeweils unabhängig voneinander gewählt werden.

R1, R2, R3 stehen unabhängig voneinander für eine Gruppierung $-(CH_2-CH_2-O)_v-H$, oder für eine Gruppierung der allgemeinen Formel (EFA-II).

Die Indexzahl v kann für eine ganze Zahl von 0 bis 100 stehen.

Hierbei besteht die Maßgabe, dass mindestens einer der Reste R1, R2, R3 für eine Gruppierung der allgemeinen Formel (EFA-II) steht.

Da mindestens einer der Reste R1, R2, R3 für eine Gruppierung der allgemeinen Formel (EFA-II) steht, ist gewährleistet, dass die Verbindung der Formel (EFA-I) in Form eines Esters aus Glycerin und einer $C_{12}$-$C_{24}$-Fettsäure vorliegt.

Weiter besteht die Maßgabe, dass mindestens eine der Indexzahlen für v, n und/oder q für eine Zahl ungleich 0 stehen.

Steht nur der Rest R1 für eine Verbindung der Formel (EFA-II), dann stehen die anderen beiden Reste R2, und R3 unabhängig voneinander jeweils für eine Gruppierung $-(CH_2-CH_2-O)_v-H$.

In jedem der beiden Resten R2 und R3 kann die Indexzahl v unabhängig vom anderen Rest gewählt werden. Durch die Maßgabe, dass mindestens eine Indexzahl v, n und/oder q für eine Zahl ungleich 0 steht, ist gewährleistet, dass die Verbindung der Formel (EFA-I) in Form eines ethoxylierten Fettsäureesters vorliegt.

Stehen zwei Reste aus R1, R2, und R3 für eine Verbindung der Formel (EFA-II), dann steht der verbleibende Rest für eine Gruppierung $-(CH_2-CH_2-O)_v-H$. In jeder der Formeln (EFA-II) können die Indexzahlen unabhängig von den Indexzahlen der jeweils anderen Formel (EFA-II) gewählt werden. Der verbleibende Rest aus R1, R2 und R3 steht für die Gruppierung $-(CH_2-CH_2-O)_v-H$.

Durch die Maßgabe, dass mindestens eine Indexzahl v, n und/oder q für eine Zahl ungleich 0 steht, ist gewährleistet, dass die Verbindung der Formel (EFA-I) in Form eines ethoxylierten Fettsäureesters vorliegt.

Stehen alle drei Reste R1, R2, und R3 für eine Verbindung der Formel (EFA-II), dann kann natürlich keiner dieser Reste für eine Gruppierung $-(CH_2-CH_2-O)_v-H$ stehen. In jeder der Formeln (EFA-II) können die Indexzahlen unabhängig von den Indexzahlen der jeweils anderen Formel (EFA-II) gewählt werden. In diesem Fall existiert keine Indexzahl v. Auch in Fall gilt die Maßgabe, dass mindestens eine Indexzahl v, n und/oder q für eine Zahl ungleich 0 steht. Da die Indexzahl v im Rahmen dieser Ausführungsform jedoch nicht existiert, bedeutet dies, dass mindestens eine der vorhandenen Indexzahlen n und/oder q für eine Zahl ungleich 0 stehen muss.

Durch die Vorgabe, dass mindestens einer der Indexzahlen v, n und/oder q für eine Zahl ungleich 0 stehen, ist gewährleistet, dass in der Verbindung der Formel (EFA-I) mindestens ein Addukt mit Ethylenoxid vorliegt, so dass im Molekül mindestens eine Gruppierung *-CH2-CH2-O-* vorhanden ist.

Die Indexzahlen n, m, o, p, q, r und s können in jeder Struktureinheit der Formel (EFA-II) unabhängig von den anderen Struktureinheiten gewählt werden.

Die Indexzahl n steht für eine ganze Zahl von 0 bis 100.

Die Indexzahl r steht für eine ganze Zahl von 1 bis 100.

Die Zahlen n und r geben in jeder Struktureinheit der Formel (EFA-II) die Anzahl an Ethylenoxid-Eineheiten an.

Die Indexzahl m steht für eine ganze Zahl von 1 bis 8.

Die Indexzahl o steht für eine ganze Zahl von 0 bis 3.

Die Indexzahl p steht für eine ganze Zahl von 1 bis 8.

Die Indexzahl q steht für die Zahl 0 oder 1.

Die Indexzahl s steht für eine ganze Zahl von 1 bis 8.

Die Indexzalen m, o, p, q und s definieren die Länge der $C_{12}$-$C_{24}$-Fettsäure, wobei auch hier die erfüllt sein muss, dass alle Indexzahlen so gewählt werden, dass sich die Struktureinheit der Formel (EFA-II) von einer $C_{12}$-$C_{24}$-Fettsäure ableitet.

Färbungen mit besonders hoher Farbintensität wurden erhalten, wenn ein erfindungsgemäßes Mittel auf das

Keratinmaterial appliziert wurde, welches mindestens einen ethoxylierten Fettsäureester (a3) der allgemeinen Formel (EFA-I) enthielt,
wobei

R1, R2, R3 unabhängig voneinander für eine Gruppierung der allgemeinen Formel (EFA-II) stehen,

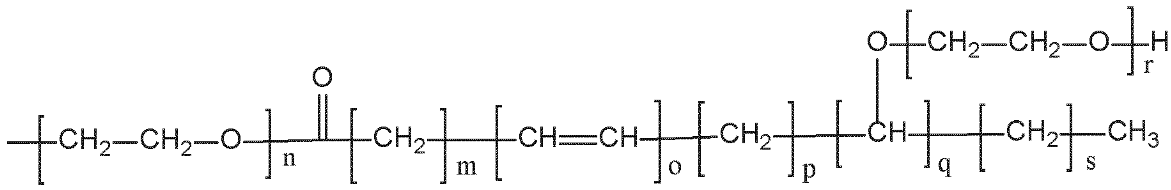

(II),

wobei

- n für die Zahl von 0 steht
- m für eine ganze Zahl von 1 bis 8 steht,
- o für die Zahl 0 oder 1, bevorzugt für die Zahl 0, steht,
- p für eine ganze Zahl von 1 bis 8 steht,
- q für die Zahl 1 steht,
- r für eine ganze Zahl von 1 bis 100, und
- s für eine ganze Zahl von 1 bis 8 steht.

Im Rahmen einer weiteren ganz besonders bevorzugten Ausführungsform ist ein erfindungsgemäßes Mittel dadurch gekennzeichnet, dass es
(a3) mindestens einen ethoxylierten Fettsäureester der allgemeinen Formel (EFA-I) enthält
wobei

R1, R2, R3 unabhängig voneinander für eine Gruppierung der allgemeinen Formel (EFA-II) stehen,

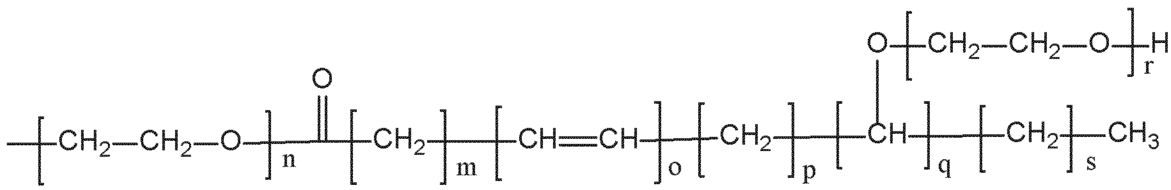

(EFA-II),

wobei

- n für die Zahl von 0 steht
- m für eine ganze Zahl von 1 bis 8 steht,
- o für die Zahl 0 oder 1, bevorzugt für die Zahl 0, steht,
- p für eine ganze Zahl von 1 bis 8 steht,
- q für die Zahl 1 steht,
- r für eine ganze Zahl von 1 bis 100, und
- s für eine ganze Zahl von 1 bis 8 steht.

Die Indexzalen m, o, p, q und s definieren wieder die Länge der $C_{12}$-$C_{24}$-Fettsäure, wobei auch hier die Maßgabe besteht, dass alle Indexzahlen so gewählt werden, dass sich die Struktureinheit der Formel (EFA-II) von einer $C_{12}$-$C_{24}$-Fettsäure ableitet.
Steht o für die Zahl 0, so ist die Summe aus m, p, q und s eine ganze Zahl von 10 bis 22.
Steht o für die Zahl 1, so ist die Summe aus m, p, q und s eine ganze Zahl von 8 bis 20.
Es haben sich die ethoxylierten Fettsäureester (a3) zur Lösung der erfindungsgemäßen Aufgabenstellung als ganz besonders gut geeignet erweisen, die ausgewählt sind aus der Gruppe aus PEG-5 Hydrogenated Castor Oil, PEG-10 Hydrogenated Castor Oil, PEG-20 Hydrogenated Castor Oil, PEG-30 Hydrogenated Castor Oil, PEG-40

Hydrogenated Castor Oil, PEG-50 Hydrogenated Castor Oil, PEG-60 Hydrogenated Castor Oil, PEG-70 Hydrogenated Castor Oil, PEG-80 Hydrogenated Castor Oil, PEG-90 Hydrogenated Castor Oil, PEG-100 Hydrogenated Castor Oil, PEG-5 Castor Oil, PEG-10 Castor Oil, PEG-20 Castor Oil, PEG-30 Castor Oil, PEG-40 Castor Oil, PEG-50 Castor Oil, PEG-60 Castor Oil, PEG-70 Castor Oil, PEG-70 Castor Oil, PEG-80 Castor Oil, PEG-90 Castor Oil und PEG-100 Castor Oil.

Im Rahmen einer weiteren ganz besonders bevorzugten Ausführungsform ist ein erfindungsgemäßes Mittel dadurch gekennzeichnet, dass es

(a3) mindestens einen ethoxylierten Fettsäureester enthält, der ausgewählt ist aus der Gruppe aus PEG-5 Hydrogenated Castor Oil, PEG-10 Hydrogenated Castor Oil, PEG-20 Hydrogenated Castor Oil, PEG-30 Hydrogenated Castor Oil, PEG-40 Hydrogenated Castor Oil, PEG-50 Hydrogenated Castor Oil, PEG-60 Hydrogenated Castor Oil, PEG-70 Hydrogenated Castor Oil, PEG-80 Hydrogenated Castor Oil, PEG-90 Hydrogenated Castor Oil, PEG-100 Hydrogenated Castor Oil, PEG-5 Castor Oil, PEG-10 Castor Oil, PEG-20 Castor Oil, PEG-30 Castor Oil, PEG-40 Castor Oil, PEG-50 Castor Oil, PEG-60 Castor Oil, PEG-70 Castor Oil, PEG-70 Castor Oil, PEG-80 Castor Oil, PEG-90 Castor Oil und PEG-100 Castor Oil.

Am allermeisten ist PEG-60 Hydrogenated Castor Oil (a3) bevorzugt.

PEG-60 Hydrogenated Castor Oil trägt die CAS-Nummer 61788-85-0 und kann alternativ auch als Castor oil, hydrogenated, ethoxylated (60 EO) bezeichnet werden. Unter dem Handelsnamen Eumulgin CO 60 kann es beispielsweise von der Firma BASF kommerziell erworben werden.

Im Rahmen einer weiteren ganz besonders bevorzugten Ausführungsform ist ein erfindungsgemäßes Mittel dadurch gekennzeichnet, dass es

(a3) mindestens einen ethoxylierten Fettsäureester der allgemeinen Formel (EFA-I) enthält

wobei

R1, R2, R3 unabhängig voneinander für ein Wasserstoffatom, für eine Gruppierung - $(CH_2-CH_2-O)_v$-H, oder für eine Gruppierung der allgemeinen Formel (EFA-II) stehen,

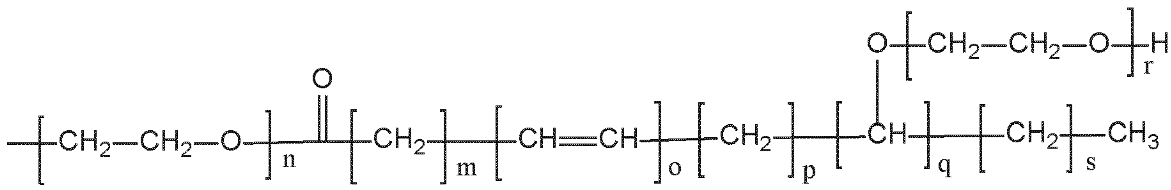

(EFA-II),

wobei

- v für eine ganze Zahl von 0 bis 100 steht,
- n für eine ganze Zahl von 0 bis 100, bevorzugt für eine ganze Zahl von 1 bis 10, steht
- m für eine ganze Zahl von 1 bis 8 steht,
- o für die Zahl 0 steht,
- p für eine ganze Zahl von 1 bis 8 steht,
- q für die Zahl 0 steht,
- s für eine ganze Zahl von 1 bis 8 steht,

mit der Maßgabe, dass

- mindestens einer der Reste R1, R2, R3 für eine Gruppierung der allgemeinen Formel (II) steht, und dass
- mindestens eine der Indexzahlen für v und/oder n für eine Zahl ungleich 0 stehen.

Steht nur der Rest R1 für eine Verbindung der Formel (EFA-II), dann stehen die anderen beiden Reste R2, und R3 unabhängig voneinander jeweils für eine Gruppierung -$(CH_2-CH_2-O)_v$-H.

In jedem der beiden Resten R2 und R3 kann die Indexzahl v unabhängig vom anderen Rest gewählt werden. Da die Indexzahl q für 0 steht, existiert keine Gruppierung, welche die Indexzahl r trägt. Durch die Maßgabe, dass mindestens eine Indexzahl v und/oder n für eine Zahl ungleich 0 steht, ist gewährleistet, dass die Verbindung der Formel (EFA-I) in Form eines ethoxylierten Fettsäureesters vorliegt.

Stehen zwei Reste aus R1, R2, und R3 für eine Verbindung der Formel (EFA-II), dann steht der verbleibende Rest für eine Gruppierung -$(CH_2-CH_2-O)_v$-H. In jeder der Formeln (EFA-II) können die Indexzahlen unabhängig von den Indexzahlen der jeweils anderen Formel (EFA-II) gewählt werden. Der verbleibende Rest aus R1, R2 und R3 steht für die

Gruppierung $-(CH_2-CH_2-O)_v-H$.

Da die Indexzahl q für 0 steht, existiert keine Gruppierung, welche die Indexzahl r trägt. Durch die Maßgabe, dass mindestens eine Indexzahl v und/oder n für eine Zahl ungleich 0 steht, ist gewährleistet, dass die Verbindung der Formel (EFA-I) in Form eines ethoxylierten Fettsäureesters vorliegt.

Stehen alle drei Reste R1, R2, und R3 für eine Verbindung der Formel (EFA-II), dann kann natürlich keiner dieser Reste für eine Gruppierung $-(CH_2-CH_2-O)_v-H$ stehen. In jeder der Formeln (EFA-II) können die Indexzahlen unabhängig von den Indexzahlen der jeweils anderen Formel (EFA-II) gewählt werden. In diesem Fall existiert keine Indexzahl v. Da die Indexzahl q für 0 steht, existiert auch keine Gruppierung, welche die Indexzahlr trägt. Auch in Fall gilt die Maßgabe, dass mindestens eine Indexzahl v und/oder n für eine Zahl ungleich 0 steht. Da die Indexzahl v im Rahmen dieser Ausführungsform jedoch nicht existiert, bedeutet dies, dass mindestens eine der vorhandenen Indexzahlen n für eine Zahl ungleich 0 stehen muss.

Die Indexzahl v steht für eine ganze Zahl von 0 bis 100.

Im Rahmen dieser Ausführungsform steht n für eine ganze Zahl von 0 bis 100, bevorzugt steht n für eine ganze Zahl von 0 bis 10. Die Indexzahl q steht für die Zahl 0.

Die Anzahl der Einheiten *-CH2-CH2-O-* in jeder strukturellen Einheit (EFA-II) wird daher durch die jeweilige Indexzahl n vorgegeben.

Die Indexzahlen o und q stehn beide für die Zahl 0. Damit wird die Länge der $C_{12}$-$C_{24}$-Fettsäure durch die Indexzahlen m, p und s definiert, wobei auch hier wieder die Voraussetzung gilt, dass alle Indexzahlen so gewählt werden, dass sich die Struktureinheit der Formel (EFA-II) von einer $C_{12}$-$C_{24}$-Fettsäure ableitet.

Hierbei gilt, dass die Summe aus m, p und s eine ganze Zahl von 10 bis 22 ist.

Weiterhin haben sich zur Lösung der erfindungsgemäßen Aufgabenstellung die die ethoxlierten Fettsäureester (a3) als ganz besonders gut geeignet erweisen, die ausgewählt ist aus der Gruppe aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, PEG-7 Glycerylcocoat, PEG-7 Glyceryldilaurat, PEG-7 Glyceryldimyristat, PEG-7 Glyceryldipalmitat, PEG-7 Glyceryldistearat, PEG-7 Glyceryldicaprylat, PEG-7 Glyceryldicaprat, PEG-10 Glyceryldilaurat, PEG-10 Glyceryldimyristat, PEG-10 Glyceryldipalmitat, PEG-10 Glyceryldistearat, PEG-10 Glyceryldicaprylat, PEG-10 Glyceryldicaprat, PEG-5 Glyceryldilaurat, PEG-5 Glyceryldimyristat, PEG-5 Glyceryldipalmitat, PEG-5 Glyceryldistearat, PEG-5 Glyceryldicaprylat, PEG-5 Glyceryldicaprat, PEG-7 Glycerylcocoat, PEG-7 Glyceryltrilaurat, PEG-7 Glyceryltrimyristat, PEG-7 Glyceryltripalmitat, PEG-7 Glyceryltristearat, PEG-7 Glyceryltricaprylat, PEG-7 Glyceryltricaprat, PEG-10 Glyceryltrilaurat, PEG-10 Glyceryltrimyristat, PEG-10 Glyceryltripalmitat, PEG-10 Glyceryltristearat, PEG-10 Glyceryltricaprylat, PEG-10 Glyceryltricaprat, PEG-5 Glyceryltrilaurat, PEG-5 Glyceryltrimyristat, PEG-5 Glyceryltripalmitat, PEG-5 Glyceryltristearat, PEG-5 Glyceryltricaprylat, PEG-5 Glyceryltricaprat, PEG-7 Glycerylcocoat und deren Mischungen.

Im Rahmen einer weiteren ganz besonders bevorzugten Ausführungsform ist ein erfindungsgemäßes Mittel dadurch gekennzeichnet, dass es

(a3) mindestens einen ethoxylierten Fettsäureester enthält, der ausgewählt ist aus der Gruppe aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, PEG-7 Glycerylcocoat, PEG-7 Glyceryldilaurat, PEG-7 Glyceryldimyristat, PEG-7 Glyceryldipalmitat, PEG-7 Glyceryldistearat, PEG-7 Glyceryldicaprylat, PEG-7 Glyceryldicaprat, PEG-10 Glyceryldilaurat, PEG-10 Glyceryldimyristat, PEG-10 Glyceryldipalmitat, PEG-10 Glyceryldistearat, PEG-10 Glyceryldicaprylat, PEG-10 Glyceryldicaprat, PEG-5 Glyceryldilaurat, PEG-5 Glyceryldimyristat, PEG-5 Glyceryldipalmitat, PEG-5 Glyceryldistearat, PEG-5 Glyceryldicaprylat, PEG-5 Glyceryldicaprat, PEG-7 Glycerylcocoat, PEG-7 Glyceryltrilaurat, PEG-7 Glyceryltrimyristat, PEG-7 Glyceryltripalmitat, PEG-7 Glyceryltristearat, PEG-7 Glyceryltricaprylat, PEG-7 Glyceryltricaprat, PEG-10 Glyceryltrilaurat, PEG-10 Glyceryltrimyristat, PEG-10 Glyceryltripalmitat, PEG-10 Glyceryltristearat, PEG-10 Glyceryltricaprylat, PEG-10 Glyceryltricaprat, PEG-5 Glyceryltrilaurat, PEG-5 Glyceryltrimyristat, PEG-5 Glyceryltripalmitat, PEG-5 Glyceryltristearat, PEG-5 Glyceryltricaprylat, PEG-5 Glyceryltricaprat, PEG-7 Glycerylcocoat und deren Mischungen.

Explizit ganz besonders bevorzugt ist PEG-7 Glyceryl Cocoate, welches die CAS-Nummer 68553-02-6 trägt und beispielsweise unter dem Handelsnamen Cetiol HE von der Firma BASF kommerziell erworben werden kann.

Die ethoxylierten Fettsäureester (a3) werden besonders bevorzugt in bestimmten Mengenbereichen im erfindungsgemäßen Mittel eingesetzt.

Besonders gute Ergebnisse wurden erhalten, wenn das Mittel - bezogen auf das Gesamtgewicht des Mittels - ein

oder mehere ethoxylierte Fettsäureester (a3) in einer Gesamtmenge von 0,1 bis 20,0 Gew.-%, bevorzugt 0,5 bis 15,0 Gew.-%, weiter bevorzugt von 1,0 bis 10,0 Gew.-% und ganz besonders bevorzugt von 4,0 bis 8,0 Gew.-% enthielt.

Im Rahmen einer weiteren bevorzugten Ausführungsform ist ein erfindungsgemäßes Mittel dadurch gekennzeichnet, dass es - bezogen auf das Gesamtgewicht des Mittels - ein oder mehere ethoxylierte Fettsäureester (a3) in einer Gesamtmenge von 0,1 bis 20,0 Gew.-%, bevorzugt 0,5 bis 15,0 Gew.-%, weiter bevorzugt von 1,0 bis 10,0 Gew.-% und ganz besonders bevorzugt von 4,0 bis 8,0 Gew.-% enthält.

Fettbestandteile im Mittel

Als weiteren optionalen Bestandteil kann das erfindungsgemäße Mittel zusätzlich auch noch mindestens einen Fettbestandteil enthalten.

Es hat sich herausgestellt, dass der Einsatz mindestens eines Fettbestandteils dazu führt, dass das Mittel in Form einer Emulsion vorliegt, welche die optimale Viskosität besitzt und sich auch im Hinblick auf die Verbesserung der Farbintensität als vorteilhaft herausgestellt hat.

Bei den Fettbestandteilen handelt es sich um hydrophobe Substanzen, die in Gegenwart von Wasser unter Ausbildung von Mizell-Systemen Emulsionen formen können.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um ethoxylierte, noch um polyoxyalkylierte noch um polyglycerylierte Verbindungen.

Ganz besonders bevorzugt werden die im Mittelenthaltenen Fettbestandteile (a4) ausgewählt aus der Gruppe der $C_{12}$-$C_{24}$-Fettalkohole, der $C_{12}$-$C_{24}$-Fettsäuretriglyceride, der $C_{12}$-$C_{24}$-Fettsäure-monoglyceride, der $C_{12}$-$C_{24}$-Fettsäurediglyceride und/oder der Kohlenwasserstoffe.

Im Rahmen einer weiteren bevorzugten Ausführungsform ist ein erfindungsgemäßes Mittel dadurch gekennzeichnet, dass es einen oder mehrere Fettbestandteile aus der Gruppe der $C_{12}$-$C_{24}$-Fettalkohole, der $C_{12}$-$C_{24}$-Fettsäuretriglyceride, der $C_{12}$-$C_{24}$-Fettsäuremonoglyceride, der $C_{12}$-$C_{24}$-Fettsäurediglyceride und/oder der Kohlenwasserstoffe enthält.

Als ganz besonders bevorzugte Fettbestandteile werden in diesem Zusammenhang die Bestandteile aus der Gruppe der $C_{12}$-$C_{24}$-Fettalkohole, der $C_{12}$-$C_{24}$-Fettsäuretriglyceride, der $C_{12}$-$C_{24}$-Fettsäuremonoglyceride, der $C_{12}$-$C_{24}$-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{24}$-Fettalkoholen kann es sich um gesättigte, ein- oder mehrfach ungesättigte, lineare oder verzweigte Fettalkohole mit 12 bis 24 C-Atomen handeln.

Beispiele für bevorzugte lineare, gesättigte $C_{12}$-$C_{24}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9E)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13Z)-Docos-13-en-1-ol) und/oder Brassidylalkohol ((13E)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Im Rahmen einer weiteren bevorzugten Ausführungsform ist ein erfindungsgemäßes Mittel dadurch gekennzeichnet, dass es einen oder mehrere $C_{12}$-$C_{24}$-Fettalkohole aus der Gruppe aus

Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9*Z*)-Octadec-9-en-1-ol (Oleylalkohol),

(9*E*)-Octadec-9-en-1-ol (Elaidylalkohol),
(9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),
Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13*E*)-Docosen-1-ol),
2-Octyl-dodecanol,
2-Hexyl-Dodecanol und/oder
2-Butyl-dodecanol enthält.

Es hat sich als ganz besonders bevorzugt erwiesen, ein oder mehrere $C_{12}$-$C_{24}$-Fettalkohole in ganz bestimmten Mengenbereichen einzusetzen.

Es ist ganz besonders bevorzugt, wenn die das Mittel - bezogen auf das Gesamtgewicht des Mittels - einen oder mehrere $C_{12}$-$C_{24}$-Fettalkohole in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin als ganz geeigneten Fettbestandteil kann das Mittel auch mindestens ein $C_{12}$-$C_{24}$-Fettsäuretriglycerid, der $C_{12}$-$C_{24}$-Fettsäuremonoglycerid und/oder $C_{12}$-$C_{24}$-Fettsäurediglycerid enthalten. Unter einem $C_{12}$-$C_{24}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{24}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{24}$-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die $C_{12}$-$C_{24}$-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem $C_{12}$-$C_{24}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Ganz besonders gute Ergebnisse wurden erhalten, wenn das Mittel mindestens ein $C_{12}$-$C_{24}$-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Im Rahmen einer weiteren Ausführungsform ist ein erfindungsgemäßes Mittel dadurch gekennzeichnet, dass es mindestens ein $C_{12}$-$C_{24}$-Fettsäuremonoglycerid enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Es hat sich als bevorzugt erwiesen, ein oder mehrere $C_{12}$-$C_{24}$-Fettsäuremono-, $C_{12}$-$C_{24}$--Fettsäuredi- und/oder $C_{12}$-$C_{24}$-Fettsäuretriglyceride in ganz bestimmten Mengenbereichen im Mittel einzusetzen.

Im Hinblick auf die Lösung der erfindungsgemäßen Aufgabenstellung hat es sich als vorteilhaft erwiesen, wenn das Mittel - bezogen auf das Gesamtgewicht des Mittels - ein oder mehrere $C_{12}$-$C_{24}$-Fettsäuremono-, $C_{12}$-$C_{24}$-Fettsäuredi- und/oder $C_{12}$-$C_{24}$-Fettsäuretriglyceride in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Weiterhin als ganz besonders bevorzugten Fettbestandteil kann das Mittel auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn das Mittel mindestens einen Kohlenwasserstoff enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Mittel dadurch gekennzeichnet, dass es mindestens einen Fettbestandteil aus der Gruppe der Kohlenwasserstoffe enthält.

Im Hinblick auf die Lösung der erfindungsgemäßen Aufgabenstellung hat es sich als ganz besonders bevozugt erwiesen, wenn das Mittel - bezogen auf das Gesamtgewicht des Mittels - einen oder mehrere Kohlenwasserstoffe in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthielt.

Im Rahmen einer ganz besonders bevorzugten Ausführungsform ist ein erfindungsgemäßes Mittel dadurch gekennzeichnet, dass es - bezogen auf das Gesamtgewicht des Mittels - einen oder mehrere Kohlenwasserstoffe in einer Gesamtmenge von in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthält.

Der oder die Kohlenwasserstoffe können als alleinige Fettbestandteile (a4) in den Mitteln eingesetzt werden. Es ist jedoch ebenfalls erfindungsgemäß, mindestens einen Kohlenwasserstoff in Kombination mit mindestens einem weiteren Bestandteil in die Mittel einzuarbeiten.

<u>Wassergehalt im Mittel</u>

Bei dem zuvor beschriebenen Mittel handelt es sich um ein anwendungsbereites Mittel, welches auf das keratinische Material appliziert werden kann. Dieses anwendungsbereite Mittel besitzt bevorzugt einen hohen Wasseranteil. Es hat sich herausgestellt, dass besonders die Mittel besonders gut geeignet sind, die - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthalten.

In einer weiteren explizit ganz besonders bevorzugten Ausführungsform ist ein erfindungsgemäßes Mittel dadurch gekennzeichnet, dass es - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthält.

weitere optionale Inhalttstoffe im Mittel

Zusätzlich zu den bereits beschriebenen erfindungswesentlichen Bestandteilen (a1) bis (a3) kann das Mittel zusätzlich auch noch weitere optionale Inhaltsstoffe enthalten.

So kann das Mittel beispielsweise ein filmbildendes Polymer enthalten. Das filmbildende Polymer kann zum Beispiel ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/EthylenCopolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Weitere geeignete filmbildende Polymere können augewählt sein aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Es haben sich insbesondere die filmbildenden Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer $C_1$-$C_{20}$-Alkylgruppe, einer Arylgruppe oder einer C2-C10-Hydroxyalkylgruppe.

Weitere filmbildende Polymere können ausgewählt sein aus den Homo- oder Copolymeren von Isooctyl-(meth) acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere geeignete anionische Copolymere sind beispielsweise Copolymere aus Acrylsäure, Methacrylsäure oder deren $C_1$-$C_6$-Alkylestern, wie sie unter der INCI-Deklaration Acrylates Copolymere vertrieben werden. Ein geeignetes Handelsprodukt ist beispielsweise Aculyn® 33 der Firma Rohm & Haas. Weiterhin bevorzugt sind aber auch Copolymere aus Acrylsäure, Methacrylsäure oder deren $C_1$-$C_6$-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche Polymere sind beispielsweise Aculyn® 22 (Acrylates/Steareth-20 Methacrylate Copolymer), Aculyn® 28 (Acrylates/Beheneth-25 Methacrylate Copolymer), Structure 2001®(Acryla-tes/Steareth-20 Itaconate Copolymer), Structure 3001®(Acrylates/Ceteth-20 Itaconate Copolymer), Structure Plus® (Acrylates/Aminoacrylates C10-30 Alkyl PEG-20 Itaconate Copolymer), Carbopol® 1342, 1382, Ultrez 20, Ultrez 21 (Acrylates/C10-30 Alkyl Acrylate Crosspolymer), Synthalen W 2000® (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin geeignet sind die Copolymere Octylacrylamid/acrylates/ butylaminoethyl-methacrylate copolymer, wie es beispielsweise unter den Handelsnamen AMPHOMER® ou LOVOCRYL® 47 von NATIONAL STARCH kommerziell vertrieben wird, oder auch die Copolymere von Acrylates/Octylacrylamide die unter den Handelsnamen DERMACRYL® LT und DERMACRYL® 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Wenn im erfindungsgemäßen Mittel prinzipiell auch sowohl anionische als auch kationische und/oder nichtionische Polymere eingesetzt werden können, so hat es sich als ganz besonders bevorzugt erwiesen, weitere ionische Verbindungen nicht oder nur in geringen Mengen einzusetzen. Mit anderen Worten konnte insbesondere dann eine besonders starke Verbesserung der Farbintensität erzielt werden, wenn das Mittel eine vorwiegend nichtionische Basis darstellte und daher kationische und anionische Polymere entweder gar nicht oder nur in sehr geringen Mengen enthielt. Aus diesem Grund ist hat es sich als ganz besonders bevorzugt herausgestellt, wenn der Gesamtgehalt aller im Mittel enthaltenen anionischen Polymer unterhalb von 0,1 Gew.-% liegt. Weiterhin hat es als ganz besonders bevorzugt herausgestellt, wenn der Gesamtgehalt aller im Mittel enthaltenen kationischen Polymeren unterhalb von 0,1 Gew.-% liegt. Der Mengenanteil an katonischen bzw. anionischem Polymer ist hierbei jeweils auf das Gesamtgewicht des Mittels bezogen.

In einer weiteren ganz besonders bevorzugten Ausführungsform ist ein erfindungsgemäßes Mittel dadurch gekennzeichnet, dass - bezogen auf das Gesamtgewicht des Mittels -

- der Gesamtgehalt aller im Mittel enthaltenen anionischen Polymere unterhalb von 0,1 Gew.-% liegt, und
- der Gesamtgehalt aller im Mittel enthaltenen kationischen Polymere unterhalb von 0,1 Gew.-% liegt.

Neben den zuvor beschriebenen nichtionischen Tensiden können die Mittel prinzipiell zusätzlich auch noch ein oder mehrere geladene Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -$COO^{(-)}$ - oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcar boxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Beispiele für ampholytische Tenside sind das N-Kokosalkylaminopropionat, das Kokosacylaminoethylaminopropionat und das $C_{12}$ - $C_{18}$ - Acylsarcosin.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind

- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Wenn im erfindungsgemäßen Mittel prinzipiell auch sowohl anionische als auch kationische und/oder nichtionische Tenside eingesetzt werden können, so hat es sich als ganz besonders bevorzugt erwiesen, weitere ionische Verbindungen nicht oder nur in geringen Mengen einzusetzen. Mit anderen Worten konnte insbesondere dann eine besonders starke Verbesserung der Farbintensität erzielt werden, wenn das Mittel eine vorwiegend nichtionische Basis darstellte und daher kationische und anionische Tenside entweder gar nicht oder nur in sehr geringen Mengen enthielt. Aus diesem Grund ist hat es sich als ganz besonders bevorzugt herausgestellt, wenn der Gesamtgehalt aller im Mittel enthaltenen anionischen Tenside unterhalb von 0,1 Gew.-% liegt. Weiterhin hat es als ganz besonders bevorzugt herausgestellt, wenn der Gesamtgehalt aller im Mittel enthaltenen kationischen Tenside unterhalb von 0,1 Gew.-% liegt. Der Mengenanteil an katonischen bzw. anionischem Tensid ist hierbei jeweils auf das Gesamtgewicht des Mittels bezogen.

In einer weiteren ganz besonders bevorzugten Ausführungsform ist ein erfindungsgemäßes Mittel dadurch gekennzeichnet, dass - bezogen auf das Gesamtgewicht des Mittels -

- der Gesamtgehalt aller im Mittel enthaltenen anionischen Tenside unterhalb von 0,1 Gew.-% liegt, und
- der Gesamtgehalt aller im Mittel enthaltenen kationischen Tenside unterhalb von 0,1 Gew.-% liegt.

Die Mittel können ferner auch noch weitere Wirk-, Hilfs- und Zusatzstoffe enthalten, wie beispielsweise Lösungsmittel, Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

pH-Wert der Mittel

Die pH-Werte des erfindungsgemäßen Mittels wird bevorzugt auf einen neutral bis alkalischen pH-Wert eingestellt. Ganz besonders bevorzugt besitzt das Mittel einen alkalischen pH-Wert im Bereich von 7,0 bis 11,5 bevorzugt von 8,0 bis 11,0, und besonders bevorzugt von 8,5 bis 10,5. Unter basischen Bedingungen kann das aminofunktionalisierte Silikonpolymer (a1) besonders gut und ohne Protonierung gelöst bzw. dispergiert werden.

Im Rahmen einer weiteren bevorzugten Ausführungsform ist ein erfindungsgemäßes Mittel dadurch gekennzeichnet, dass es einen pH-Wert von 7,0 bis 11,5 bevorzugt von 8,0 bis 11,0, und besonders bevorzugt von 8,5 bis 10,5 besitzt.

Zur Einstellung des gewünschten pH-Wertes kann das Mittel (a) und/oder (b) mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

Verfahren zum Färben von Keratinmaterial

Die zuvor beschriebenen Mittel lassen sich hervorragend in Verfahren zum Färben von keratinischem Material, insbesondere von menschlichen Haaren einsetzen.

Ein zweiter Gegenstand der vorliegenden Erfindung ist daher ein Verfahren zum Färben von keratinischem Material, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,
(2) Einwirken des Färbemittels auf dem keratinischen Material und
(3) Ausspülen des Färbemittels mit Wasser.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.

In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das

(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

Im Anschluss an das Einwirken der Anwendungsmischung auf das Keratinmaterial wird diese schließlich in Schritt (3) des Verfahrens mit Wasser ausgespült.

Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen des Mittels in Schritt (3) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das

(3) Ausspülen des Färbemittels ausschließlich mit Wasser.

Betreffend die weiteren bevorzugten Ausführungsformen der erfindungsgemäßen Verfahrens gilt *mutatis mutantis* das zum erfindungsgemäßen Mittel gesagte.

Beispiele

1. Formulierungen

Es wurden die folgenden Formulierungen hergestellt (alle Angaben, sofern nichts anderes angegeben ist, in Gew.-%):

| Färbemittel | (V1) | (E1) | (E2) |
|---|---|---|---|
| Cetylalkohol | 6,0 | 6,0 | 6,0 |
| $C_{12}$-$C_{18}$-Fettalkohole (Lorol techn.) | 6,0 | 6,0 | 6,0 |
| Ceteareth-30 (Cetearylalkohol, ethoxylated 30 EO) | 6,0 | --- | --- |
| PEG-7 Glyceryl Cocoat | --- | 6,0 | --- |
| PEG-60 Hydrogenated Castor Oil | --- | --- | 6,0 |
| Lavanya Zuni (organisches Pigment, Neelikon Red, 111P0200, CI 12490) | 1,0 | 1,0 | 1,0 |
| Dow Corning 2-8566 (Siloxanes and Silicones, 3-[(2-Aminoethyl)amino]-2-methylpropyl Me, Di-Me-Siloxane" | 2,5 | 2,5 | 2,5 |
| Ammoniak (25%ige wässrige Lösung) | 0,20 | 0,20 | 0,20 |
| Wasser | ad 100 | ad 100 | ad 100 |

2. Anwendung

Nach der Herstellung wurde das jeweilige Mittel (V1, E1 und E2) auf Haarsträhnen (Kerling, Euronaturhaar weiß, Flottenverhältnis: 1 g Mittel (E1) pro g Haarsträhne) appliziert. Das Mittel wurde für drei Minuten einwirken gelassen. Im Anschluss daran wurden die Haarsträhne gründlich (1 Minute) mit Wasser ausgewaschen, getrocknet und dann farbmetrisch mit einem Farbmessgerät der Firma Datacolor, Typ Spectraflash 450 vermessen.

Der für die Beurteilung der Farbintensität herangezogene dE-Wert ergibt sich aus den am jeweiligen Strähnenteil gemessenen L*a*b*-Farbmesswerten wie folgt:

$$dE = [\ (L_i - L_0)^2 + (a_i - a_0)^2 + (b_i - b_0)]^{1/2}$$

$L_0$, $a_0$ und $b_0$ = Messwerte der Vergleichsfärbung (V1)
$L_i$, $a_i$ und $b_i$ = Messwerte der erfindungsgemäßen Färbung (E)

Die Buntheit einer Färbung (Chroma) berechnet sich nach der Formel

$$C = \sqrt{a^2 + b^2}$$

Je größer der C-Werst ist, desto höher ist die Buntheit einer Färbung.

Der L-Wert gibt die Helligkeit einer Färbung an. Je niedriger der L-Wert ist, desto dunkler und intensiver ist die Färbung

| Mittel | L | a | b | Chroma C | dE zum Vergleich |
|---|---|---|---|---|---|
| Vergleich (V1) | 40,55 | 37,65 | 8,22 | 38,54 | |
| Erfindung (E1) | 28,96 | 47,52 | 18,02 | 50,82 | 18,10 |
| Erfindung (E2) | 29,18 | 45,76 | 15,19 | 48,21 | 15,61 |

Mit den erfindungsgemäßen Mitteln wurden dunklere, intensivere Färbungen (niedrigere L-Werte) und eine höhere Buntheit (größerer C-Wert) gemessen.

## Patentansprüche

1. Mittel zum Färben von keratinischem Material, insbesondere menschlichen Haaren, enthaltend

(a1) mindestens ein aminofunktionalisiertes Silikonpolymer, das Struktureinheiten der Formel (Si-I) und der Formel (Si-II) umfasst

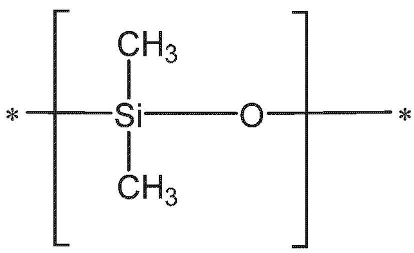
(Si-I)

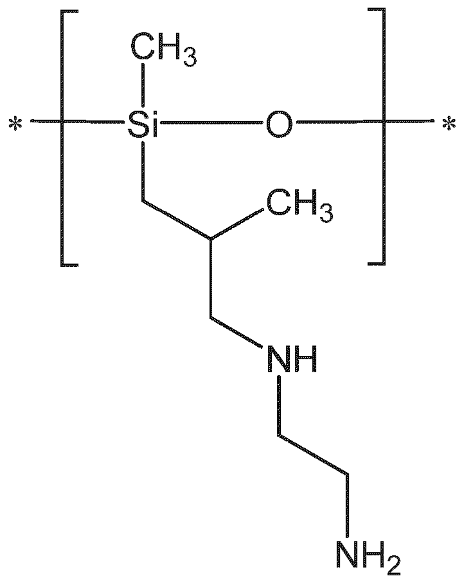
(Si-II),

und
(a2) mindestens eine farbgebende Verbindung aus der Gruppe der Pigmente, und

(a3) mindestens ein Anlagerungsprodukt von Ethylenoxid an Ester gebildet aus $C_{12}$-$C_{24}$-Fettsäuren und aliphatischen $C_1$-$C_{12}$-Alkoholen.

2. Mittel nach Anspruch 1, **dadurch gekennzeichnet, dass** es - bezogen auf das Gesamtgewicht des Mittels - ein oder mehrere aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

3. Mittel nach einem der Ansprüche 1 bis 2, **dadurch gekennzeichnet, dass** es mindestens eine farbgebende Verbindung (a2) aus der Gruppe der anorganischen Pigmente enthält, die bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

4. Mittel nach einem der Ansprüche 1 bis 3, **dadurch gekennzeichnet, dass** es mindestens eine farbgebende Verbindung (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

5. Mittel nach einem der Ansprüche 1 bis 4, **dadurch gekennzeichnet, dass** es - bezogen auf das Gesamtgewicht des Mittels (a) - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

6. Mittel nach einem der Ansprüche 1 bis 5, **dadurch gekennzeichnet, dass** es
(a3) mindestens ein Anlagerungsprodukt von Ethylenoxid an einen Ester enthält, der durch Veresterung von einer, zwei oder drei $C_{12}$-$C_{24}$-Fettsäuren mit Glycerin erhalten wird.

7. Mittel nach einem der Ansprüche 1 bis 6, **dadurch gekennzeichnet, dass** es
(a3) mindestens einen ethoxylierten Fettsäureester der allgemeinen Formel (EFA-I) enthält

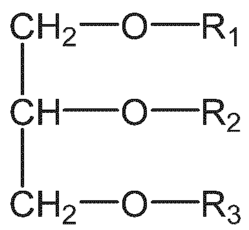

(EFA-I)

wobei

R1, R2, R3 unabhängig voneinander für eine Gruppierung $-(CH_2-CH_2-O)_v-H$, oder für eine Gruppierung der allgemeinen Formel (EFA-II) stehen,

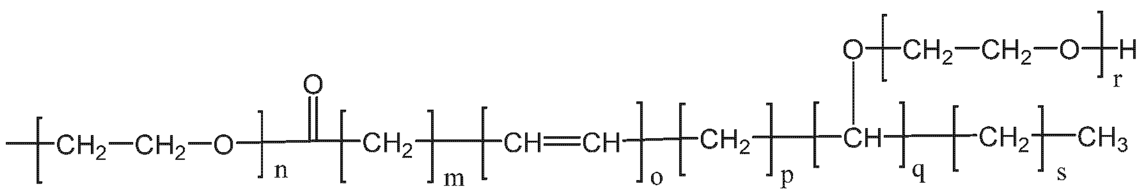

(EFA-II),

wobei

v für eine ganze Zahl von 0 bis 100 steht,
n für eine ganze Zahl von 0 bis 100 steht
m für eine ganze Zahl von 1 bis 8 steht,
o für eine ganze Zahl von 0 bis 3 steht,
p für eine ganze Zahl von 1 bis 8 steht,
q für die Zahl 0 oder 1 steht,
r für eine ganze Zahl von 1 bis 100, und
s für eine ganze Zahl von 1 bis 8 steht,

mit der Maßgabe, dass

- mindestens einer der Reste R1, R2, R3 für eine Gruppierung der allgemeinen Formel (EFA-II) steht, und dass
- mindestens eine der Indexzahlen für v, n und/oder q für eine Zahl ungleich 0 stehen.

**8.** Mittel nach einem der Ansprüche 1 bis 7, **dadurch gekennzeichnet, dass** es (a3) mindestens einen ethoxylierten Fettsäureester der allgemeinen Formel (EFA-I) enthält wobei

R1, R2, R3 unabhängig voneinander für eine Gruppierung der allgemeinen Formel (EFA-II) stehen,

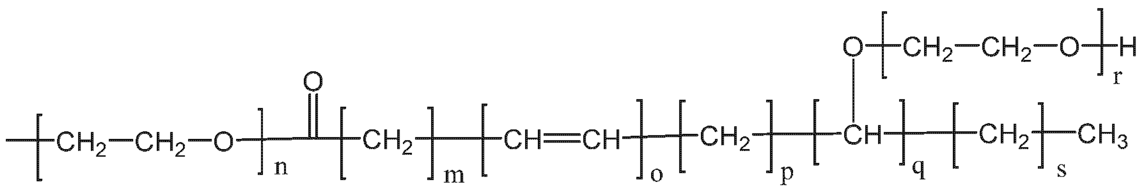

(EFA-II),

wobei

n für die Zahl von 0 steht
m für eine ganze Zahl von 1 bis 8 steht,
o für die Zahl 0 oder 1, bevorzugt für die Zahl 0, steht,
p für eine ganze Zahl von 1 bis 8 steht,
q für die Zahl 1 steht,
r für eine ganze Zahl von 1 bis 100, und
s für eine ganze Zahl von 1 bis 8 steht.

**9.** Mittel nach einem der Ansprüche 1 bis 8, **dadurch gekennzeichnet, dass** es (a3) mindestens einen ethoxylierten Fettsäureester enthält, der ausgewählt ist aus der Gruppe aus PEG-5 Hydrogenated Castor Oil, PEG-10 Hydrogenated Castor Oil, PEG-20 Hydrogenated Castor Oil, PEG-30 Hydrogenated Castor Oil, PEG-40 Hydrogenated Castor Oil, PEG-50 Hydrogenated Castor Oil, PEG-60 Hydrogenated Castor Oil, PEG-70 Hydrogenated Castor Oil, PEG-80 Hydrogenated Castor Oil, PEG-90 Hydrogenated Castor Oil, PEG-100 Hydrogenated Castor Oil, PEG-5 Castor Oil, PEG-10 Castor Oil, PEG-20 Castor Oil, PEG-30 Castor Oil, PEG-40 Castor Oil, PEG-50 Castor Oil, PEG-60 Castor Oil, PEG-70 Castor Oil, PEG-70 Castor Oil, PEG-80 Castor Oil, PEG-90 Castor Oil und PEG-100 Castor Oil.

**10.** Mittel nach einem der Ansprüche 1 bis 9, **dadurch gekennzeichnet, dass** es (a3) mindestens einen ethoxylierten Fettsäureester der allgemeinen Formel (EFA-I) enthält wobei

R1, R2, R3 unabhängig voneinander für ein Wasserstoffatom, für eine Gruppierung $-(CH_2-CH_2-O)_v-H$, oder für eine Gruppierung der allgemeinen Formel (EFA-II) stehen,

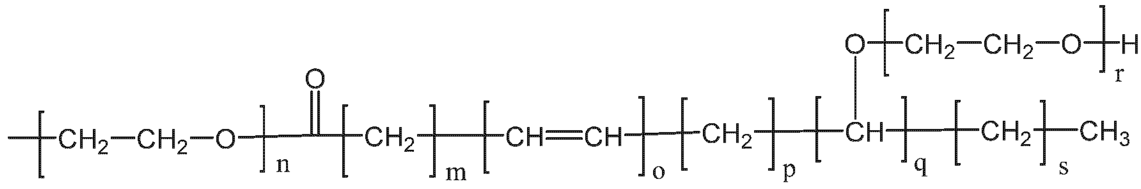

(EFA-II),

wobei

- v für eine ganze Zahl von 0 bis 100 steht,
- n für eine ganze Zahl von 0 bis 100, bevorzugt für eine ganze Zahl von 1 bis 10, steht
- m für eine ganze Zahl von 1 bis 8 steht,
- o für die Zahl 0 steht,
- p für eine ganze Zahl von 1 bis 8 steht,
- q für die Zahl 0 steht,
- s für eine ganze Zahl von 1 bis 8 steht,

mit der Maßgabe, dass

- mindestens einer der Reste R1, R2, R3 für eine Gruppierung der allgemeinen Formel (II) steht, und dass
- mindestens eine der Indexzahlen für v und/oder n für eine Zahl ungleich 0 stehen.

**11.** Mittel nach einem der Ansprüche 1 bis 10, **dadurch gekennzeichnet, dass** es (a3) mindestens einen ethoxylierten Fettsäureester enthält, der ausgewählt ist aus der Gruppe aus PEG-7 Glycerylmonolaurat, PEG-7 Glycerylmonomyristat, PEG-7 Glycerylmonopalmitat, PEG-7 Glycerylmonostearat, PEG-7 Glycerylmonocaprylat, PEG-7 Glycerylmonocaprat, PEG-10 Glycerylmonolaurat, PEG-10 Glycerylmonomyristat, PEG-10 Glycerylmonopalmitat, PEG-10 Glycerylmonostearat, PEG-10 Glycerylmonocaprylat, PEG-10 Glycerylmonocaprat, PEG-5 Glycerylmonolaurat, PEG-5 Glycerylmonomyristat, PEG-5 Glycerylmonopalmitat, PEG-5 Glycerylmonostearat, PEG-5 Glycerylmonocaprylat, PEG-5 Glycerylmonocaprat, PEG-7 Glycerylcocoat, PEG-7 Glyceryldilaurat, PEG-7 Glyceryldimyristat, PEG-7 Glyceryldipalmitat, PEG-7 Glyceryldistearat, PEG-7 Glyceryldicaprylat, PEG-7 Glyceryldicaprat, PEG-10 Glyceryldilaurat, PEG-10 Glyceryldimyristat, PEG-10 Glyceryldipalmitat, PEG-10 Glyceryldistearat, PEG-10 Glyceryldicaprylat, PEG-10 Glyceryldicaprat, PEG-5 Glyceryldilaurat, PEG-5 Glyceryldimyristat, PEG-5 Glyceryldipalmitat, PEG-5 Glyceryldistearat, PEG-5 Glyceryldicaprylat, PEG-5 Glyceryldicaprat, PEG-7 Glycerylcocoat, PEG-7 Glyceryltrilaurat, PEG-7 Glyceryltrimyristat, PEG-7 Glyceryltripalmitat, PEG-7 Glyceryltristearat, PEG-7 Glyceryltricaprylat, PEG-7 Glyceryltricaprat, PEG-10 Glyceryltrilaurat, PEG-10 Glyceryltrimyristat, PEG-10 Glyceryltripalmitat, PEG-10 Glyceryltristearat, PEG-10 Glyceryltricaprylat, PEG-10 Glyceryltricaprat, PEG-5 Glyceryltrilaurat, PEG-5 Glyceryltrimyristat, PEG-5 Glyceryltripalmitat, PEG-5 Glyceryltristearat, PEG-5 Glyceryltricaprylat, PEG-5 Glyceryltricaprat, PEG-7 Glycerylcocoat und deren Mischungen.

**12.** Mittel nach einem der Ansprüche 1 bis 11, **dadurch gekennzeichnet, dass** es - bezogen auf das Gesamtgewicht des Mittels - ein oder mehere ethoxylierte Fettsäureester (a3) in einer Gesamtmenge von 0,1 bis 20,0 Gew.-%, bevorzugt 0,5 bis 15,0 Gew.-%, weiter bevorzugt von 1,0 bis 10,0 Gew.-% und ganz besonders bevorzugt von 4,0 bis 8,0 Gew.-% enthält.

**13.** Mittel nach einem der Ansprüche 1 bis 12, **dadurch gekennzeichnet, dass** - bezogen auf das Gesamtgewicht des Mittels -

- der Gesamtgehalt aller im Mittel enthaltenen anionischen Tenside unterhalb von 0,1 Gew.-% liegt, und
- der Gesamtgehalt aller im Mittel enthaltenen kationischen Tenside unterhalb von 0,1 Gew.-% liegt.

**14.** Mittel nach einem der Ansprüche 1 bis 13, **dadurch gekennzeichnet, dass** - bezogen auf das Gesamtgewicht des Mittels -

- der Gesamtgehalt aller im Mittel enthaltenen anionischen Polymere unterhalb von 0,1 Gew.-% liegt, und
- der Gesamtgehalt aller im Mittel enthaltenen kationischen Polymere unterhalb von 0,1 Gew.-% liegt.

**15.** Mittel nach einem der Ansprüche 1 bis 14, **dadurch gekennzeichnet, dass** es Wasser enthält und einen pH-Wert von 7,0 bis 11,5 bevorzugt von 8,0 bis 11,0, und besonders bevorzugt von 8,5 bis 10,5 besitzt.

**16.** Verfahren zum Färben von keratinischem Material, insbesondere menschlichen Haaren, umfassend die folgenden Schritte:

(1) Anwendung eines Färbemittels nach einem der Ansprüche 1 bis 15 auf dem keratinischem Material,
(2) Einwirken des Färbemittels auf dem keratinischen Material und
(3) Ausspülen des Färbemittels mit Wasser.

**17.** Verfahren nach Anspruch 16, **gekennzeichnet durch** das
(2) Einwirken des Färbemittels (a) auf dem keratinischen Material für einen Zeitraum von 30 Sekunden bis 15 Minuten, bevorzugt von 30 Sekunden bis 10 Minuten, und besonders bevorzugt von 1 bis 5 Minuten.

## Claims

**1.** An agent for dyeing keratinous material, in particular human hair, containing

(a1) at least one amino-functionalized silicone polymer comprising structural units of formula (Si-I) and formula (Si-II)

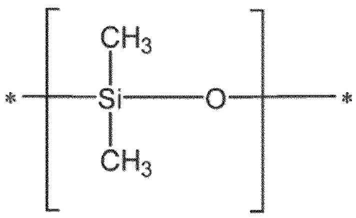
(Si-I)

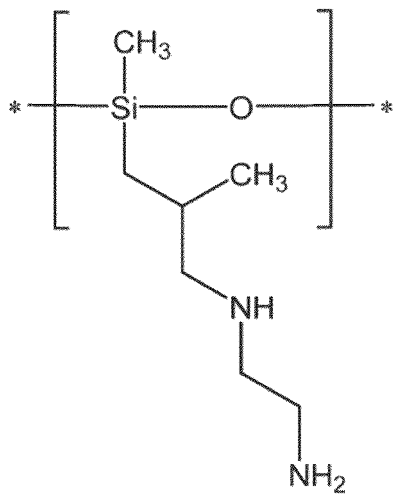
(Si-II),

and
(a2) at least one coloring compound from the group of pigments, and
(a3) at least one addition product of ethylene oxide to esters formed from $C_{12}$-$C_{24}$ fatty acids and aliphatic $C_1$-$C_{12}$ alcohols.

**2.** The agent according to claim 1, **characterized in that** it contains, based on the total weight of the agent, one or more amino-functionalized silicone polymers (a1) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

**3.** The agent according to one of claims 1 to 2, **characterized in that** it contains at least one coloring compound (a2) from the group of inorganic pigments, preferably selected from the group of colored metal oxides, metal hydroxides, metal oxide hydrates, silicates, metal sulfides, complex metal cyanides, metal sulfates, bronze pigments and/or from mica-based colored pigments which are coated with at least one metal oxide and/or a metal oxychloride.

**4.** The agent according to one of claims 1 to 3, **characterized in that** it contains at least one coloring compound (a2) from the group of organic pigments, preferably selected from the group of carmine, quinacridone, phthalocyanine, sorghum, blue pigments with the Color Index numbers CI 42090, CI 69800, CI 69825, CI 73000, CI 74100 or CI 74160, yellow pigments with the Color Index numbers CI 11680, CI 11710, CI 15985, CI 19140, CI 20040, CI 21100, CI 21108, CI 47000 or CI 47005, green pigments with the Color Index numbers CI 61565, CI 61570 or CI 74260, orange

pigments with the Color Index numbers CI 11725, CI 15510, CI 45370 or CI 71105, and red pigments with the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

5. The agent according to one of claims 1 to 4, **characterized in that** it contains, based on the total weight of the agent (a), one or more pigments (a2) in a total amount from 0.01 to 10.0 wt.%, preferably from 0.1 to 5.0 wt.%, more preferably from 0.2 to 2.5 wt.%, and very particularly preferably from 0.25 to 1.5 wt.%.

6. The agent according to one of claims 1 to 5, **characterized in that** it (a3) contains at least one addition product of ethylene oxide to an ester, which is obtained by esterification of one, two or three $C_{12}$-$C_{24}$ fatty acids with glycerin.

7. The agent according to one of claims 1 to 6, **characterized in that** it (a3) contains at least one ethoxylated fatty acid ester of general formula (EFA-I)

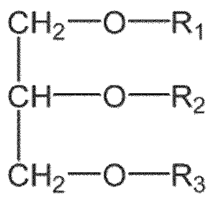
(EFA-I)

wherein
R1, R2, R3 independently represent a $-(CH_2-CH_2-O)_v-H$ group, or a group of general formula (EFA-II),

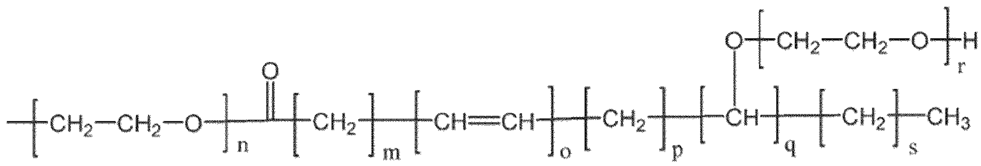

(EFA-II),

wherein

v represents an integer from 0 to 100,
n represents an integer from 0 to 100,
m represents an integer from 1 to 8,
o represents an integer from 0 to 3,
p represents an integer from 1 to 8,
q represents the number 0 or 1,
r represents an integer from 1 to 100, and
s represents an integer from 1 to 8,

with the proviso that

- at least one of the functional groups R1, R2, R3 represents a group of general formula (EFA-II), and that
- at least one of the index numbers for v, n and/or q represents a number other than 0.

8. The agent according to one of claims 1 to 7, **characterized in that** it
(a3) contains at least one ethoxylated fatty acid ester of general formula (EFA-I)
wherein
R1, R2, R3 independently represent a group of general formula (EFA-II),

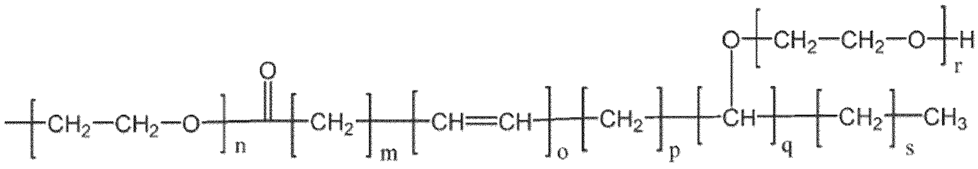

(EFA-II),

wherein

n represents the number 0,
m represents an integer from 1 to 8,
o represents the number 0 or 1, preferably the number 0,
p represents an integer from 1 to 8,
q represents the number 1,
r represents an integer from 1 to 100, and
s represents an integer from 1 to 8.

**9.** The agent according to one of claims 1 to 8, **characterized in that** it
(a3) contains at least one ethoxylated fatty acid ester selected from the group of PEG-5 hydrogenated castor oil, PEG-10 hydrogenated castor oil, PEG-20 hydrogenated castor oil, PEG-30 hydrogenated castor oil, PEG-40 hydrogenated castor oil, PEG-50 hydrogenated castor oil, PEG-60 hydrogenated castor oil, PEG-70 hydrogenated castor oil, PEG-80 hydrogenated castor oil, PEG-90 hydrogenated castor oil, PEG-100 hydrogenated castor oil, PEG-5 castor oil, PEG-10 castor oil, PEG-20 castor oil, PEG-30 castor oil, PEG-40 castor oil, PEG-50 castor oil, PEG-60 castor oil, PEG-70 castor oil, PEG-70 castor oil, PEG-80 castor oil, PEG-90 castor oil and PEG-100 castor oil.

**10.** The agent according to one of claims 1 to 9, **characterized in that** it
(a3) contains at least one ethoxylated fatty acid ester of general formula (EFA-I), wherein
R1, R2, R3 independently represent a hydrogen atom, a $-(CH_2-CH_2-O)_V-H$ group, or a group of general formula (EFA-II),

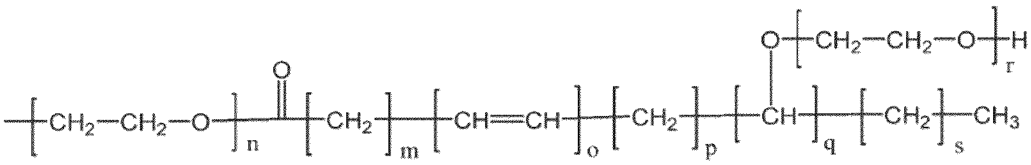

(EFA-II),

wherein

v represents an integer from 0 to 100,
n represents an integer from 0 to 100, preferably an integer from 1 to 10,
m represents an integer from 1 to 8,
o represents the number 0,
p represents an integer from 1 to 8,
q represents the number 0,
s represents an integer from 1 to 8,

with the proviso that

- at least one of the functional groups R1, R2, R3 represents a group of general formula (II), and that
- at least one of the index numbers for v and/or n represents a number other than 0.

**11.** The agent according to one of claims 1 to 10, **characterized in that** it
(a3) contains at least one ethoxylated fatty acid ester selected from the group of PEG-7 glyceryl monolaurate, PEG-7 glyceryl monomyristate, PEG-7 glyceryl monopalmitate, PEG-7 glyceryl monostearate, PEG-7 glyceryl monocaprylate, PEG-7 glyceryl monocaprate, PEG-10 glyceryl monolaurate, PEG-10 glyceryl monomyristate, PEG-10 glyceryl monopalmitate, PEG-10 glyceryl monostearate, PEG-10 glyceryl monocaprylate, PEG-10 glyceryl monocaprate, PEG-5 glyceryl monolaurate, PEG-5 glyceryl monomyristate, PEG-5 glyceryl monopalmitate, PEG-5 glyceryl monostearate, PEG-5 glyceryl monocaprylate, PEG-5 glyceryl monocaprate, PEG-7 glyceryl cocoate, PEG-7 glyceryl dilaurate, PEG-7 glyceryl dimyristate, PEG-7 glyceryl dipalmitate, PEG-7 glyceryl distearate, PEG-7 glyceryl dicaprylate, PEG-7 glyceryl dicaprate, PEG-10 glyceryl dilaurate, PEG-10 glyceryl dimyristate, PEG-10 glyceryl dipalmitate, PEG-10 glyceryl distearate, PEG-10 glyceryl dicaprylate, PEG-10 glyceryl dicaprate, PEG-5 glyceryl dilaurate, PEG-5 glyceryl dimyristate, PEG-5 glyceryl dipalmitate, PEG-5 glyceryl distearate, PEG-5 glyceryl dicaprylate, PEG-5 glyceryl dicaprate, PEG-7 glyceryl cocoate, PEG-7 glyceryl trilaurate, PEG-7 glyceryl trimyristate, PEG-7 glyceryl tripalmitate, PEG-7 glyceryl tristearate, PEG-7 glyceryl tricaprylate, PEG-7 glyceryl tricaprate, PEG-10 glyceryl trilaurate, PEG-10 glyceryl trimyristate, PEG-10 glyceryl tripalmitate, PEG-10 glyceryl tristearate, PEG-10 glyceryl tricaprylate, PEG-10 glyceryl tricaprate, PEG-5 glyceryl trilaurate, PEG-5 glyceryl trimyristate, PEG-5 glyceryl tripalmitate, PEG-5 glyceryl tristearate, PEG-5 glyceryl tricaprylate, PEG-5 glyceryl tricaprate, PEG-7 glyceryl cocoate and mixtures thereof.

**12.** The agent according to one of claims 1 to 11, **characterized in that** it contains, based on the total weight of the agent, one or more ethoxylated fatty acid esters (a3) in a total amount from 0.1 to 20.0 wt.%, preferably from 0.5 to 15.0 wt.%, more preferably from 1.0 to 10.0 wt.%, and very particularly preferably from 4.0 to 8.0 wt.%.

**13.** The agent according to one of claims 1 to 12, **characterized in that,** based on the total weight of the agent,

- the total content of all anionic surfactants contained in the agent is below 0.1 wt.%, and
- the total content of all cationic surfactants contained in the agent is below 0.1 wt.%.

**14.** The agent according to one of claims 1 to 13, **characterized in that,** based on the total weight of the agent,

- the total content of all anionic polymers contained in the agent is below 0.1 wt.%, and
- the total content of all cationic polymers contained in the agent is below 0.1 wt.%.

**15.** The agent according to one of claims 1 to 14, **characterized in that** it contains water and has a pH in the range of 7.0 to 11.5, preferably of 8.0 to 11.0, and particularly preferably of 8.5 to 10.5.

**16.** A method for dyeing keratinous material, in particular human hair, comprising the following steps:

(1) applying a dyeing agent according to one of claims 1 to 15 to the keratinous material,
(2) allowing the dyeing agent to act on the keratinous material, and
(3) rinsing out the dyeing agent using water.

**17.** The method according to claim 16, **characterized by**
(2) allowing the dyeing agent (a) to act on the keratinous material for a period of 30 seconds to 15 minutes, preferably 30 seconds to 10 minutes, and particularly preferably 1 to 5 minutes.

## Revendications

**1.** Agent permettant la coloration d’une matière kératinique, en particulier de cheveux humains, contenant

(a1) au moins un polymère de silicone aminofonctionnalisé qui comprend des motifs structuraux de formule (Si-I) et de formule (Si-II)

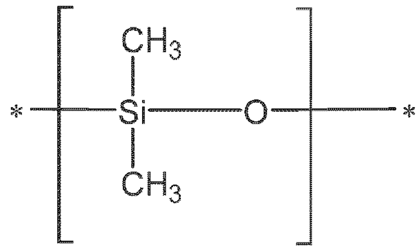 (Si-I) 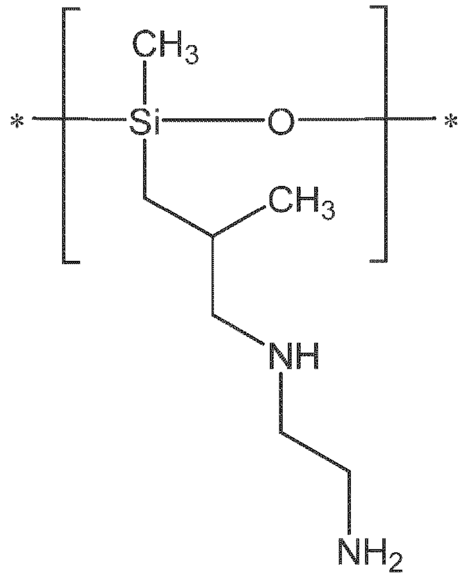 (Si-II),

et

(a2) au moins un composé colorant du groupe des pigments, et

(a3) au moins un produit d'addition d'oxyde d'éthylène à des esters formés à partir d'acides gras en $C_{12}$-$C_{24}$ et d'alcools aliphatiques en $C_1$-$C_{12}$.

2. Agent selon la revendication 1, **caractérisé en ce qu'il** contient, par rapport au poids total de l'agent, un ou plusieurs polymères de silicone aminofonctionnalisés (a1) en une quantité totale allant de 0,1 à 8,0 % en poids, de préférence de 0,2 à 5,0 % en poids, plus préférablement de 0,3 à 3,0 % en poids et de manière tout particulièrement préférée de 0,4 à 2,5 % en poids.

3. Procédé selon l'une des revendications 1 à 2, **caractérisé en ce qu'**il contient au moins un composé colorant (a2) du groupe des pigments inorganiques qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica recouverts d'au moins un oxyde métallique et/ou oxychlorure métallique.

4. Agent selon l'une des revendications 1 à 3, **caractérisé en ce qu'**il contient au moins un composé colorant (a2) du groupe des pigments organiques qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

5. Agent selon l'une des revendications 1 à 4, **caractérisé en ce qu'**il contient, par rapport au poids total de l'agent (a), un ou plusieurs pigments (a2) en une quantité totale allant de 0,01 à 10,0 % en poids, de préférence de 0,1 à 5,0 % en poids, plus préférablement de 0,2 à 2,5 % en poids et de manière tout particulièrement préférée de 0,25 à 1,5 % en poids.

6. Agent selon l'une des revendications 1 à 5, **caractérisé en ce qu'**il
(a3) contient au moins un produit d'addition d'oxyde d'éthylène à un ester obtenu par estérification d'un, de deux ou de trois acides gras en $C_{12}$-$C_{24}$ avec du glycérol.

7. Agent selon l'une des revendications 1 à 6, **caractérisé en ce qu'**il
(a3) contient au moins un ester d'acide gras éthoxylé de formule générale (EFA-I)

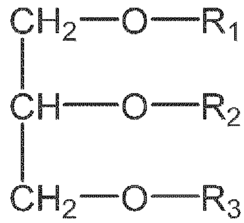

(EFA-I)

où

R1, R2, R3 représentent indépendamment les uns des autres un groupement $-(CH_2-CH_2-O)_V-H$, ou un groupement de formule générale (EFA-II),

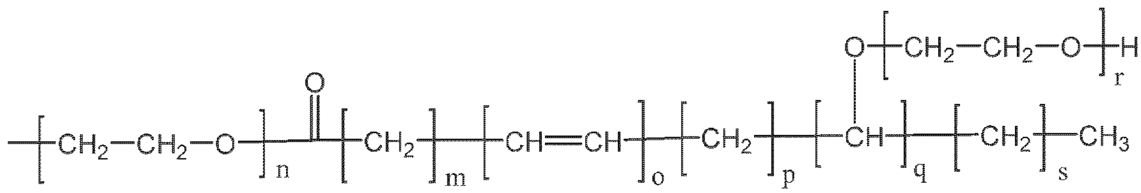

(EFA-II),

où

v représente un nombre entier allant de 0 à 100,
n représente un nombre entier allant de 0 à 100,
m représente un nombre entier allant de 1 à 8,
o représente un nombre entier allant de 0 à 3,
p représente un nombre entier allant de 1 à 8,
q représente le nombre 0 ou 1,
r représente un nombre entier allant de 1 à 100, et
s représente un nombre entier allant de 1 à 8,

à condition que

- au moins l'un des radicaux R1, R2, R3 représente un groupement de formule générale (EFA-II), et que
- au moins l'un des indices pour v, n et/ou q représente un nombre différent de 0.

**8.** Agent selon l'une des revendications 1 à 7, **caractérisé en ce qu'**il
(a3) contient au moins un ester d'acide gras éthoxylé de formule générale (EFA-I)
où
R1, R2, R3 représentent indépendamment les uns des autres un groupement de formule générale (EFA-II),

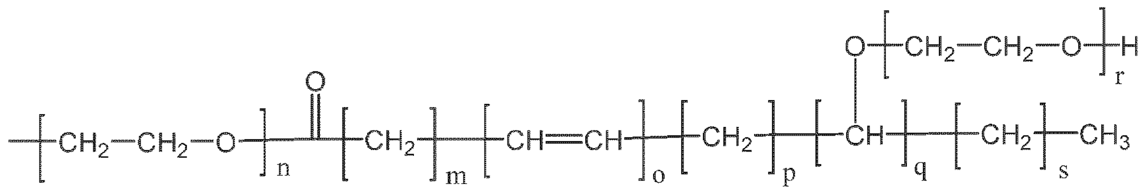

(EFA-II),

où

n représente le nombre 0

m représente un nombre entier allant de 1 à 8,
o représente le nombre 0 ou 1, de préférence le nombre 0,
p représente un nombre entier allant de 1 à 8,
q représente le nombre 1,
r représente un nombre entier allant de 1 à 100, et
s représente un nombre entier allant de 1 à 8.

**9.** Agent selon l'une des revendications 1 à 8, **caractérisé en ce qu'**il
(a3) contient au moins un ester d'acide gras éthoxylé qui est choisi dans le groupe constitué d'huile de ricin hydrogénée PEG-5, huile de ricin hydrogénée PEG-10, huile de ricin hydrogénée PEG-20, huile de ricin hydrogénée PEG-30, huile de ricin hydrogénée PEG-40, huile de ricin hydrogénée PEG-50, huile de ricin hydrogénée PEG-60, huile de ricin hydrogénée PEG-70, huile de ricin hydrogénée PEG-80, huile de ricin hydrogénée PEG-90, huile de ricin hydrogénée PEG-100, huile de ricin PEG-5, huile de ricin PEG-10, huile de ricin PEG-20, huile de ricin PEG-30, huile de ricin PEG-40, huile de ricin PEG-50, huile de ricin PEG-60, huile de ricin PEG-70, huile de ricin PEG-70, huile de ricin PEG-80, huile de ricin PEG-90 et huile de ricin PEG-100.

**10.** Agent selon l'une des revendications 1 à 9, **caractérisé en ce qu'**il
(a3) contient au moins un ester d'acide gras éthoxylé de formule générale (EFA-I) où
R1, R2, R3 représentent indépendamment les uns des autres un atome d'hydrogène, un groupement $-(CH_2-CH_2-O)_V-H$, ou un groupement de formule générale (EFA-II),

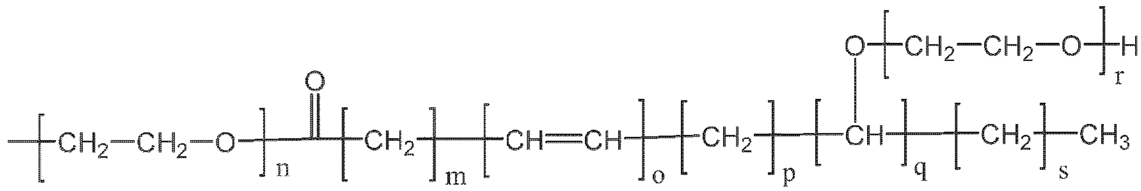

(EFA-II),

où

v représente un nombre entier allant de 0 à 100,
n représente un entier allant de 0 à 100, de préférence un nombre entier allant de 1 à 10,
m représente un nombre entier allant de 1 à 8,
o représente le nombre 0,
p représente un nombre entier allant de 1 à 8,
q représente le nombre 0,
s représente un nombre entier allant de 1 à 8,

à condition que

- au moins l'un des radicaux R1, R2, R3 représente un groupement de formule générale (II), et que
- au moins l'un des indices pour v et/ou n représente un nombre différent de 0.

**11.** Agent selon l'une des revendications 1 à 10, **caractérisé en ce qu'**il
(a3) contient au moins un ester d'acide gras éthoxylé choisi dans le groupe constitué de monolaurate de glycéryle PEG-7, monomyristate de glycéryle PEG-7, monopalmitate de glycéryle PEG-7, monostéarate de glycéryle PEG-7, monocaprylate de glycéryle PEG-7, monocaprate de glycéryle PEG-7, monolaurate de glycéryle PEG-10, monomyristate de glycéryle PEG-10, monopalmitate de glycéryle PEG-10, monostéarate de glycéryle PEG-10, monocaprylate de glycéryle PEG-10, monocaprate de glycéryle PEG-10, monolaurate de glycéryle PEG-5, monomyristate de glycéryle PEG-5, monopalmitate de glycéryle PEG-5, monostéarate de glycéryle PEG-5, monocaprylate de glycéryle PEG-5, monocaprate de glycéryle PEG-5, cocoate de glycéryle PEG-7, dilaurate de glycéryle PEG-7, dimyristate de glycéryle PEG-7, dipalmitate de glycéryle PEG-7, distéarate de glycéryle PEG-7, dicaprylate de glycéryle PEG-7, dicaprate de glycéryle PEG-7, dilaurate de glycéryle PEG-10, dimyristate de glycéryle PEG-10, dipalmitate de glycéryle PEG-10, distéarate de glycéryle PEG-10, dicaprylate de glycéryle PEG-10, dicaprate de

glycéryle PEG-10, dilaurate de glycéryle PEG-5, dimyristate de glycéryle PEG-5, dipalmitate de glycéryle PEG-5, distéarate de glycéryle PEG-5, dicaprylate de glycéryle PEG-5, dicaprate de glycéryle PEG-5, cocoate de glycéryle PEG-7, trilaurate de glycéryle PEG-7, trimyristate de glycéryle PEG-7, tripalmitate de glycéryle PEG-7, tristéarate de glycéryle PEG-7, tricaprylate de glycéryle PEG-7, tricaprate de glycéryle PEG-7, trilaurate de glycéryle PEG-10, trimyristate de glycéryle PEG-10, tripalmitate de glycéryle PEG-10, tristéarate de glycéryle PEG-10, tricaprylate de glycéryle PEG-10, tricaprate de glycéryle PEG-10, trilaurate de glycéryle PEG-5, trimyristate de glycéryle PEG-5, tripalmitate de glycéryle PEG-5, tristéarate de glycéryle PEG-5, tricaprylate de glycéryle PEG-5, tricaprate de glycéryle PEG-5, cocoate de glycéryle PEG-7 et leurs mélanges.

**12.** Agent selon l'une des revendications 1 à 11, **caractérisé en ce qu'**il contient, par rapport au poids total de l'agent, un ou plusieurs polymères de silicone aminofonctionnalisés (a3) en une quantité totale allant de 0,1 à 20,0 % en poids, de préférence de 0,5 à 15,0 % en poids, plus préférablement de 1,0 à 10,0 % en poids et de manière tout particulièrement préférée de 4,0 à 8,0 % en poids.

**13.** Agent selon l'une des revendications 1 à 12, **caractérisé en ce que,** par rapport au poids total de l'agent,

- la teneur totale de tous les tensioactifs anioniques contenus dans l'agent est inférieure à 0,1 % en poids, et
- la teneur totale de tous les tensioactifs cationiques contenus dans l'agent est inférieure à 0,1 % en poids.

**14.** Agent selon l'une des revendications 1 à 13, **caractérisé en ce que,** par rapport au poids total de l'agent,

- la teneur totale de tous les polymères anioniques contenus dans l'agent est inférieure à 0,1 % en poids, et
- la teneur totale de tous les polymères cationiques contenus dans l'agent est inférieure à 0,1 % en poids.

**15.** Agent selon l'une des revendications 1 à 14, **caractérisé en ce qu'**il contient de l'eau et possède un pH allant de 7,0 à 11,5, de préférence de 8,0 à 11,0 et de manière particulièrement préférée de 8,5 à 10,5.

**16.** Procédé permettant la coloration d'une matière kératinique, en particulier de cheveux humains, comprenant les étapes suivantes :

(1) application d'un agent de coloration selon l'une des revendications 1 à 15 sur la matière kératinique,
(2) attente de l'action de l'agent de coloration sur la matière kératinique et
(3) rinçage de l'agent de coloration avec de l'eau.

**17.** Procédé selon la revendication 16, **caractérisé par**
(2) l'attente de l'action de l'agent de coloration (a) sur la matière kératinique pendant une durée allant de 30 secondes à 15 minutes, de préférence de 30 secondes à 10 minutes, et de manière particulièrement préférée de 1 à 5 minutes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2015090804 A1
- WO 2014044602 A2
- DE 102014221536 A1
- WO 2018206453 A1


### In der Beschreibung aufgeführte Nicht-Patentliteratur


- *CHEMICAL ABSTRACTS*, 106842-44-8
- *CHEMICAL ABSTRACTS*, 75-21-8
- *CHEMICAL ABSTRACTS*, 68553-02-6